(19) Europäisches Patentamt
European Patent Office
Office européen des brevets

(11) **EP 2 861 984 B1**

(12) **EUROPEAN PATENT SPECIFICATION**

(45) Date of publication and mention
of the grant of the patent:
**18.09.2019 Bulletin 2019/38**

(51) Int Cl.:
***G01N 33/497*** (2006.01)   ***C12Q 1/00*** (2006.01)
***G01N 21/39*** (2006.01)   ***G01N 21/35*** (2014.01)
***C12M 1/34*** (2006.01)

(21) Application number: **13737424.5**

(22) Date of filing: **17.06.2013**

(86) International application number:
**PCT/IL2013/050520**

(87) International publication number:
**WO 2013/190550 (27.12.2013 Gazette 2013/52)**

(54) **METHOD AND SYSTEM FOR USE IN MONITORING BIOLOGICAL MATERIAL**

VERFAHREN UND SYSTEM ZUR ÜBERWACHUNG VON BIOLOGISCHEM MATERIAL

PROCÉDÉ ET SYSTÈME S'UTILISANT DANS LA SURVEILLANCE D'UNE MATIÈRE BIOLOGIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.06.2012 US 201261660744 P
11.11.2012 US 201213674034**

(43) Date of publication of application:
**22.04.2015 Bulletin 2015/17**

(73) Proprietor: **VAYU Sense AG
80333 München (DE)**

(72) Inventors:
• **AXELROD, Noel
97842 Jerusalem (IL)**

• **NUTTMAN, David
8008 Zürich (CH)**
• **SHIMONI, Moria
4951935 Petah Tikva (IL)**

(74) Representative: **Schatt, Markus F. et al
Stolmár & Partner
Blumenstrasse 17
80331 München (DE)**

(56) References cited:
**WO-A2-2006/047336   WO-A2-2008/053507
WO-A2-2012/001633   US-A- 4 889 992
US-A- 5 155 019**

EP 2 861 984 B1

**Description**

The present invention relates to a method for measuring the concentrations of at least one metabolic gas and system for use in detection of micro organisms or living cells.

Monitoring the live biological activity in a biological material is needed in various industries, for example in the medical field for monitoring microorganisms contaminants in blood/blood-components, in food and beverages (F&B) industries, and in pharmaceutical industries for example for monitoring fermentation processes.

The conventional techniques for monitoring a biological activity in a biological material generally include direct techniques such as: viable count in which a diluted samples are grown on agar medium dish; staining or microscopy; pH and glucose measurements; swirling; and optical density (OD) measurements in which a sample of the biological material taken in to a cuvette and the level of microorganisms is determined optically based on turbidity of the sampled biological material itself. Other known techniques utilize monitoring the biological activity indirectly, for example based on analysis of gases consumption (such as dissolved oxygen - $dO_2$) or accumulated (such as carbon dioxide - $CO_2$) in a sample of the biological material. In those techniques, a sample of the biological material is incubated for a period of time to allow consumption or accumulation of gases by live microorganisms contained in the sample and then the metabolic gases are analyzed chemically and/or by utilizing spectroscopic measurements.

Document US 5 155 019 A discloses a non-invasive real time system for detection of the presence of biological activity in a sealed container utilizing IR analysis of carbon dioxide with high sensitivity that can reflect biomass and biological processes state.

Document US 4 889 992 A discloses a system for non invasively and real time detecting microorganisms in culture media by means of measurement of gaseous metabolic products, wherein carbon dioxide is measured by specific IR spectroscopy in a zone of the container separated from the culture medium but still in fluidic communication with it.

For example Sussman et al., US 5,155,019, test for the presence of microorganisms in a possibly contaminated substance by inspecting a sample of the substance. The sample of the substance is transferred to a sterile culture vial that includes a growth medium for the microorganisms, the sample, medium and gas within the head space of the vial are sealed the vial is incubated, and infrared spectroscopy is used to monitor the concentration of carbon dioxide in the head space above the growth medium in the vial. The vial is positioned between a source of a beam of infrared radiation and a detector of the beam so that the beam traverses the head space.

Berndt, US 5,482,842, teaches a similar method, but uses two sources and two detectors. Bachur et al., EP 1 724 335, teach a similar method that uses one or more tunable lasers as the infrared source(s). Hoberman, US 4,889,992, teaches a vial that is adapted for use in such methods: the vial includes a passive mechanism for keeping liquid and foam from the growth medium out of the path of the infrared beam.

There is a need in the art for a novel technique for in-situ real-time and noninvasive monitoring of biological materials, enabling detection of microorganisms in a biological material, such as blood components, food products and/or biological materials used in fermentation processes, for example used in the pharmaceutical and/or food and beverages (F&B) industry. Specifically there is a need in the art for an in-situ real time technique for detection of microorganisms in a culture media utilizing measurement of gaseous products generated during bacterial proliferation.

The known techniques for detecting microorganisms in biological material by spectroscopic measurements of metabolic gas concentrations are generally not suited and/or are in-capable of in-situ real time operation. This is mainly because these techniques are invasive with respect to stored biological material that has to be inspected, i.e. they require sampling and incubation of the biological material in a separate sealed incubation container (sampling vial) which is impermeable for gases and possibly contains certain growth media. To this end, the conventional techniques utilize sampling/transferring certain amount of the biological material from a sealed container in which it is stored/maintained into a suitable sampling vial/container, which is specifically designed/selected to facilitate the spectroscopic measurements of metabolic gas(es). Conventional sampling vials used for this purpose are generally non-permeable to the metabolic gas in order to enable accumulation of high concentrations of the gas in the sampling vial. The conventional sampling vials are also specifically configured for the spectroscopic measurements (e.g. formed with specifically selected materials having high transitivity to wavelengths used in measurements). The sampled biological material is maintained in the sampling vial for sufficient time (the detection limit is only after incubation time of between 18 to 48 hours) for consumption or accumulation of relatively low or high concentrations, respectively, of gas consumed or accumulated by microorganisms contained in the biological material. As indicated above, many of the known techniques of the kind specified use incubation of a test sample while providing suitable growth conditions (e.g. providing growth medium such as agar, and/or incubating conditions/temperatures, and/or sufficient time for growth) to accelerate the microorganisms growth and accordingly accelerate production of the metabolic gases by the microorganisms contained in the sample. This is in fact because the sensitivity and/or accuracy of the known spectroscopic metabolic gas detection techniques require consumption or accumulation of relatively low or high concentrations, respectively, of the gas for the detection thereof.

The known techniques are thus not suited for *in-situ* real time non-invasive monitoring of biological activity/microorganisms in a biological material mainly because (1) they are invasive, i.e. require sampling of the biological material, (2) require specific equipment, i.e. the sampled biological material is transferred into a separate, specifically designed, sealed sampling vial (e.g. non-permeable to metabolic gases, highly transmitting for wavelengths used in spectroscopic measurements, e.g. containers made of glass/quartz), as well as typically require use of specific growth media, and (3) they are time consuming as they typically require relatively long incubation periods (e.g. 18-48 hours) of the sampled biological material for accumulation of sufficient (measurable/detectable) concentrations of the metabolic gases. Non-invasive gas monitoring methods available are not sensitive enough. However, *in-situ,* real time and non-invasive high resolution monitoring of biological activity in biological substances are needed in various fields. Specifically, such traits are needed in medical fields for handling blood and blood components for monitoring the blood/components thereof (e.g. prior to blood transfusions). Also such traits are highly needed in various fermentation processes in which sensitive, real-time monitoring of the biological activity may provide substantial increase in the yield of the fermentation process. This allows to control the growth of cells of a wide variety of microorganisms, early stage detection of cell growth, shorter R&D cycle time, provides for real time monitoring of biomass production process for increasing the yield (e.g. by determining optimal seed transfer and induction times, controlling the growth-media/biological-material composition utilizing controlled-depletion of nutrients, etc).

The present invention allows for in-situ non-invasive (without opening the container and withdrawing a portion of the biological material) real time and sensitive monitoring of a biological material, such as (but not limited to) a blood component, that can serve, intentionally or unintentionally, as a growth medium for the growth of microorganisms such as bacteria, and that is contained within a container sealed with respect to the biological material. Described is a method and device for monitoring any biological material, such as food, human or animal tissues, and cell cultures, with particular application to blood components such as platelets. The technique of the present invention allows for detecting bacterial contamination in platelets contained/stored in conventional platelet storage bags (e.g. plastic bags) based on quantitative analysis of metabolic gases, such as $CO_2$, released by bacteria inside the platelet plastic storage bag which is permeable to metabolic gases, while the plastic storage bag with the platelets remains sealed to contaminates.

The concentration of the metabolic gas in a dead space associated with the storage bag is monitored by optical/spectroscopic measurements performed according to the technique of the present invention as described in more details below. The dead space, for the purposes of the invention, is a space/region which is free of the biological material under inspection and is in fluid communication therewith. In some embodiments, the dead space is defined by a region/portion of the container above a portion thereof containing the biological material. In some other embodiments, the dead space is defined by a gas chamber/reservoir/pipe connectable to the container (e.g. in a manner maintaining the sealing of the container), so as to be in the fluid communication with biological material in the container. Such reservoir/pipe may be formed by a separate cavity configured as an extension of the dead space in the container.

The measurements of metabolic gas concentration may for example utilize spectroscopic measurements in mid-IR spectrum of light at wavelength(s) overlapping with strong absorption line(s) of a metabolic gas (e.g. CO2 or other metabolic gases). The light is transmitted through a part (dead space) of the plastic bag that is above the stored platelets, and is appropriately detected by an IR detector. The spectroscopic technique of the invention provides for determining the concentration of CO2 or other metabolic gas by measuring light absorption within the plastic bag, while allows for discarding/discriminating the absorbance of the plastic bag itself, thus enabling in-situ monitoring of the metabolic gases contained in the dead space of the bag.

The technique allows the detection of different transfusion-relevant contaminating bacterial species. This approach provides on-line measurement of respiratory gases such as CO2 at ambient atmospheric concentrations without the need for any pre-concentration or gas separation. The method is non-invasive since it does not require opening the plastic platelet bag for examination. This non-invasive bacterial detection method represents a new approach to prevent the transmission of bacterial contamination of platelets with an advantage of the method is that all measurements can be performed in real time, until right up to the time of transfusion and therefore the risk for sample errors is reduced to a minimum and the platelets' storage time is extended. Also, unlike conventional methods, the method of the present invention can be used with containers that are sealed with respect to the biological fluid, and are either impermeable or are permeable to the metabolic gas(es) being monitored. The invention includes real-time, precise monitoring of fermentation processes. In fermentation, the biological material generally added to the growth medium. Specifically for example, fermentation processes are used in the pharmaceutical industry for generating various biological substances such as: microbial cells (such as *E. coli*); microbial enzymes (catalase, amylase, protease, etc); primary metabolites (ethanol, citric acid, glutamic acid, etc); secondary metabolites (antibiotic, recombinant products: insulin, hepatitis B vaccine, interferon, etc).

The technique of the invention allows the detection of different microorganism's species including: aerobic & anaerobic; transparent & non-transparent; and pathogenic microorganisms. This approach provides on-line high resolution measurement of respiratory gases such as $CO_2$ at ambient atmospheric concentrations without the need for any incubation or gas separation. The detection method and system of the invention can apply qualitative and/or quantitative analysis and estimation of a level of biological activity of microorganisms, for example in agar plates, and monitoring of biological activity such as in the case of measuring bacterial growth in the bacterial production of pharmaceutical proteins. Microorganisms are used commercially to produce foods (such as vinegar, yogurt, cause beer and wine spoilage), antibiotics and chemicals such as ethanol. Production of some of the most important and complex pharmaceuticals such as insulin, hormones and antibodies is carried out using microorganisms (such as E. *coli*) that have been modified genetically using recombinant DNA technology. From the early stages of commercial production of recombinant proteins, the handling of recombinant cultures has been subject to challenges. One of these challenges is how to cope with the problem of instability in recombinant organisms and induction. Commercial production of products on a large scale, especially in the pharmaceutical industry using fermenters, depends heavily on the stable maintenance of the organisms. The fermentation process of recombinant bacteria needs to be precise and the bacteria concentration has to be monitored.

For clarity, in the following description, monitoring of pharmaceutical fermentation processes for generating proteins, is specifically described. However it should be understood that the present invention can be used for real time monitoring of other fermentation processes in the pharmaceutical industry and/or in the F&B industries.

In fermentation, specific microorganism species are deliberately introduced into a fermentation container containing a biological material serving as growth medium. The fermentation container is kept at suitable conditions (e.g. glucose, yeast, agitation, temperature) encouraging the production of the desired biological substances/proteins by the induced microorganism species. Typically, a gas inlet is coupled to a fermentation container to supply suitable atmospheric conditions for the microorganisms' metabolism (e.g. supply of ambient air) and a gas outlet is also coupled to the fermentation container to evacuate gas which is richer in metabolic gases generated by the microorganisms' metabolism. In many cases, fermentation processes are monitored by occasionally collecting a sample from the biological material in the container and analyzing that sample (such as OD measurements) in order to determine data indicative of the amounts of microorganisms and/or the amount of the proteins in the sample, and utilize that data for controlling the fermentation process.

The amount of the biological substance, which is to be produced in the fermentation, is generally correlated with the amount/the rate of change of the amount of microorganisms in the biological material in the container. To this end, an accurate real-time monitoring of the amount of microorganisms enables accurate control over the fermentation process and provides for significantly increasing the yield of the proteins to be produced.

For example, the production of recombinant protein is correlated with an optimal induction process and microorganisms' amount. During the first stage, log (logarithmic) phase, the microorganisms is being grown to certain, very specific, well define amount in which an inducer added to the culture media. Then at a later/second stage, the recombinant bacteria stops proliferating and use their "cell energy" for the production of the recombinant protein. Problems that may occur during bacteria log phase (due to problems with nutrients, for example), lack of precision during induction and/or over grown of the bacteria may lead to lower yield. Thus, by monitoring a time profile of the amount of microorganisms and/or changes thereof, the fermentation process can be controlled to improve the yield of the generated product.

However, the conventional techniques for monitoring biomass in fermentation processes are not performed continuously and are incapable of being carried out with high sensitivity in real time, and typically involve occasional collection of samples from the fermentation container and examining these samples by techniques such as optical/critical density measurement and/or by viable counts. These results, *inter alia,* in that the optimal time points for collection of fermentation yield are often missed.

The present invention provides for an accurate real time and continuous/periodical monitoring of fermentation process by continuous/periodical monitoring of the atmospheric conditions in the container to determine data indicative of a rate of metabolic gas production by microorganisms contained therein, or a change in such rate of production, and thereby determining the amount of the microorganisms, biomass, or the rate of change in this amount. The later are processed to control the fermentation process accurately in time such as to improve the yield. For example the monitored amount of microorganisms and/or rate of change thereof may be compared with a reference data/model to control the fermentation conditions (e.g. temperatures of the fermenter, gaseous atmosphere therein, nutrients or other materials supplied during the fermentation process etc), and/or identify a time at which the fermentation process transit from the "first" (production stage) to the "second" ("proliferation stage") stages, for stopping or managing the process at this time.

The system of the present invention may be configured to continuously and/or periodically/repeatedly monitor the gas/atmosphere in the fermentation container and may be in optical communication with the gas within the container itself (e.g. above the biological material) and/or in optical communication with a gas flowing out from the fermentation container (e.g. gas being in fluid communication with the inside of the container, for example gas flowing through the gas outlet of the container). Also described is the detection of isotopologues of metabolic gases. Isotopologues are molecules that are identical except for their isotopic composition. Examples of the isotopologues of carbon dioxide are 12C1602, 13C1602, 160 12C180, 160 13C180. The natural abundance of isotopologues that contain a rare isotope is negligible in comparison to the common molecule. For example, the natural abundance of 13C1602 is 0.0111%, and the natural abundance of 180 13C180 is 10-8. Different isotopologues of the same molecules have different vibration frequencies, and thus different absorption spectra in the IR region. For example, molecules of 13C1602 have a strong absorption at 2270.29cm-1, while the absorption strength of the nearest absorption line of 12C1602 at 2277.427cm-1 is weaker by a factor of about 30 than that of 13C1602. This provides means for discrimination between different isotopologues of the same molecules by means of infrared absorption spectroscopy. In particular, a typical tunable QCL operating in continuous wavelength (CW) mode can have a beam spectral width as narrow as 0.01 cm-1. That provides means for unambiguous measurement of concentrations of isotopologues of a molecule under study in a setup as described above. Isotopologues can serve as biomarkers to trace particular metabolic processes. One example of such an application is the use of D-glucose-13C6 as a carbon based nutrition source for bacteria for checking specific metabolic processes, that in turn can be used for example to study the efficiency of the fermentation reaction of glucose for ethyl alcohol production at different stages of the fermentation process.

Thus, the present invention provides a novel system and method for in-situ nondestructive (non-invasive) real time detection of microorganisms in biological materials.

It should be understood that the term *biological material* herein relates to any material that can serve as *culture media* for growth of microorganisms. In this regards, this term includes and is not limited to blood and blood components such as platelets, F&B products and biological materials used in fermentation processes (e.g. in the F&B and/or pharmaceutical industries). Also the term *microorganisms* relates to any living organisms such as bacteria, fungi etc. The term *metabolic gas* relates to one or more gases produced or consumed by microorganism's metabolism, and may include for example gases such as carbon dioxide ($CO_2$) produced during respiration. Some not limiting examples of gases that are specifically included in this definition are carbon dioxide, oxygen, ammonia, hydrogen sulfide, methane, ethane, butane, ethylene, sulfur dioxide, carbonyl sulfide and nitric oxide. Examples of gases that are specifically excluded from this definition include argon and inert gases such as helium which do not participate in the metabolic process. For clarity, in the following description, the present invention is described specifically in relation to the carbon dioxide metabolic gas. Nevertheless it should be understood that the technique of the present invention is not limited to carbon dioxide and can also be applied to detection microorganisms based on other metabolic gases.

It should further be noted that the term *in situ* in the context of the current disclosure refers to detection of microorganisms being performed directly on metabolic gas(es) formed in the original storage / fermentation container (at times termed herein as biological material (BM) container), without sampling or opening the BM container. To this end, the detection is performed without a need to expose the biological material in the container to external microorganisms. In this regards, the term *in-situ measurement* should be interpreted broadly, as including analysis of the gas(es) directly inside the storage container/bag of the biological material, where the term *directly signifies* applied to gas(es) in a dead space being in fluid communication with the biological fluid in the storage container, namely a portion of the container itself or a reservoir/pipe non-invasively connectable to said portion of the container. For example, metabolic gas detection may be performed at the gas outlet of a fermentation container and/or at a certain gas reservoir connectable to a BM container of blood/blood-components (e.g. bag/vial) by a fluid connection that does not permit external microorganism contaminant into the BM container (e.g. utilizing a transfusion needle to connect the gas-chamber/reservoir to the BM container to allow gas flow to the reservoir without opening the BM container.

In this regards, the detection is *non-invasive* and *nondestructive* in the sense that measurement procedure does not destroy or affect in any way the biological material, and thus the biological material can still be used for its original purpose, after the detection procedure. The detection is *real-time* in the sense that no incubation period is required and the results of the detection can be obtained within a relatively short time scale (seconds or minutes).

The goal of the detection is to determinate the presence of microorganisms such as bacteria (e.g. bacteria contamination) in the biological material. Yet it can be quantitative analysis and estimation of level of biological activity of microorganisms for example in agar plates and also monitoring of biological activity of microorganisms such as in case of fermentation process.

Thus, according to the invention, there is provided a method according to claims 1-9 and a system according to claims 10-16.

According to some embodiments of the present invention the method further includes processing the data indicative of the concentration of the at least one metabolic gas utilizing equilibrium condition between a rate of generation or consumption of the at least one metabolic gas by the microorganisms and a rate of flow of the at least one metabolic gas into and/or out of the container. Then utilizing the equilibrium condition data about the microorganisms in the biological material is obtained/determined.

According to some embodiments of the present invention the spectral width of the substantially narrow spectrum of the first wavelength overlaps and exceeds a spectral width of an absorption line of said metabolic gas. Also, in some cases, the
spectral width of the first wavelength is less than a spectral distance between two spectrally adjacent absorption lines of the metabolic gas.

Moreover in some cases the spectral separation between the first and second wavelengths of the light source is substantially small such that the first and second wavelengths are characterized by same or similar transmission through predetermined materials used for containers of the biological material. In this connection, according to some embodiments the transmission of the second predetermined wavelength (being in the spectral region outside the absorption peak of the metabolic gas), is indicative of absorbance of the first wavelength by materials in the region of interest, other than the metabolic gas. These other materials typically have a spectral absorbance band substantially wider than that of the absorption peak of the metabolic gas. Thus analyzing the measured data of the transmission may include utilizing the measured transmission of the second predetermined wavelength to process the measured data of the transmission of the first predetermined wavelength, which overlaps the absorption peak. This increases the sensitivity in determination of the concentration of the metabolic gas and allows detection of the microorganisms.

The high sensitivity of detection enables determination of the concentration of the metabolic gas based on equilibrium condition between a rate of generation or consumption of the at least one metabolic gas by the microorganisms and a rate of flow of the at least one metabolic gas into and/or out of the container. Also, in cases where the region of interest at which measurements are conducted is the dead space above the portion with the biological material in the container, the high sensitivity and the use of the above mentions first and second types of wavelengths may provide for eliminating a need for *a-priory* knowledge of optical properties of the container.

The at least one metabolic gas may include one or more of the following: carbon dioxide, oxygen, ammonia, hydrogen sulfide, methane, ethane, butane, ethylene, sulfur dioxide, carbonyl sulfide and nitric oxide. In some embodiments the metabolic gas includes carbon dioxide and the at least first and second wavelengths are in a spectral regime of high absorbance by carbon dioxide. Specifically the first wavelength overlaps one of absorption peaks of carbon dioxide in that regime, and the second wavelength overlaps a transmission peak in the carbon dioxide spectrum. For example the spectral regime may be in a mid-IR regime (e.g. spectral regime of high absorbance by $CO_2$ is in the vicinity of 4.3 microns).

According to the present invention the optical measurements include spectroscopic measurements. According to the present invention, the illuminating the dead space (the region of interest to be measured) includes operating a broadly tunable coherent IR light source, for producing light of the above mentioned at least first and second wavelengths. The light is directed to propagate along a path of a certain predetermined optical path (length) through the dead space and, the method includes operating a detection module for detecting the light transmitted through the dead space. In some cases, the broadly tunable coherent IR light source is a Quantum Cascade Laser (QCL). Also in some cases, operating of the broadly tunable coherent IR light source includes modulating light intensity in the at least first and second wavelengths, and operating a lock-in amplifier (associated with the detection module) to determine the transmission of the region of interest to the modulated first and second wavelengths with high signal to noise ratio signal detection based on the modulation.

According to an embodiment of the invention the optical measurements are applied to the dead space of the container, while the container is remained sealed with respect to the biological material under measurements. To this end, in some cases the container may be permeable to the at least one metabolic gas and data about the microorganisms in the biological material are determined based on an equilibrium condition defined by diffusion of the at least one metabolic gas through walls of the container. To this end the container may be a storage container for platelets, such as a conventional platelets storage container.

According to an embodiment of the invention the optical measurements are applied to the dead space of the container, where the dead space may be defined by one or more of the following: (i) a portion of the container above the portion containing the biological material; (ii) a gas chamber configured to be connectable to the container so as to be in the fluid communication with the closed container (e.g. the gas chamber may be a gas outlet of the container; (iii) an extension of the dead space of the container by an attached reservoir transparent to the at least first and second wavelengths.

According to an embodiment of the present invention the container is configured for use in a process of fermentation. The optical measurements may be performed through one or more optical windows optically coupled to the dead space of the container. In some cases, for monitoring the fermentation process, the optical measurements and the data analysis are performed continuously or periodically. Accordingly the determined gas concentration data being thereby indicative of at least one of: (a) amount of microorganisms in said container as a function of time; and (b) a rate of change in amount of microorganisms in said container as a function of time. Thus method further includes processing the gas concentration data to monitor the fermentation process.

According to the method of the present invention for determining the concentration of at least one metabolic gas, the method includes:

(i) measuring IR transmission through the dead space in two or more wavelengths comprising the above noted first and second wavelengths. The measuring comprises: tuning a central wavelength of illuminating light each one of the two or more wavelengths; detecting IR light in the two or more wavelengths transmitted through the dead space; and generating measured data indicative of two or more intensity values comprising first and second intensity values corresponding to the light transmitted through the dead space in the first and second wavelengths for a given optical path defined by the optical system and the dead space; and

(ii) processing the measured data based on an absorption model of the at least one metabolic gas. The processing comprises determining a best fit between intensity values obtained from the absorption model and the measured intensity values, and thereby determining the concentration of the at least one metabolic gas.

It is proposed that the method further includes utilizing the concentration of the metabolic gas for estimating a degree of microbial contamination of the biological material.

According to another aspect of the present invention a system for use in detection of micro organisms in a biological material is provided. The system includes:

(a) an optical system including: a broadly tunable coherent IR light source and a detection module that includes a detector sensitive in the IR wavelength regime. The broadly tunable coherent IR light source is configured and operable for producing light in a predetermined spectrum including at least first and second predetermined wavelengths of substantially narrow spectra corresponding to respectively an absorption peak of at least one metabolic gas and a spectral region outside the absorption peak of said at least one metabolic gas. The detection module is configured for detecting light of the first and second wavelengths passing through a region of interest (e.g. being a region located in between the light source and the detection module). The detection module is configured for generating data indicative of transmission of that region of interest to the at least first and second wavelengths; and
(b) a control system connectable to the light source and to the detection module and configured and operable for carrying out the following:

- operating the light source to produce the light of at least the first and second wavelengths; the first wavelength is selected such that the detected transmission for the first wavelength provides measured data indicative of absorbance by the at least one metabolic gas in the region of interest; the second wavelength is selected such that the detected transmission for the second wavelength provides detection of measured reference data indicative of absorbance of the first wavelength by materials in the region of interest other than said at least one metabolic gas;

- receiving and analyzing the measured data and the measured reference data, and generating data indicative of the concentration of the metabolic gas in the region of interest. This thereby enables non-invasive in-situ detection of microorganisms in a biological material when located in fluid communication with the region of interest. According to an embodiment of the present invention the light source is broadly tunable light source having a tunability range of at least 2 cm$^{-1}$. The broadly tunable light source may be a Quantum Cascade Laser (QCL) with a tunability range exceeding 30 cm$^{-1}$. In an embodiment of the present invention, the detection module includes a lock-in amplifier, and the control system is adapted for operating the broadly tunable IR light source for modulating light intensity of the at least first and second wavelengths, and operating the lock-in amplifier to determine the transmission of the region of interest to the at least first and second wavelengths with high signal to noise ratio based on the modulation. To this end the optical system is configured to define illumination and detection paths for the light of said at least first and second wavelengths intersecting with the region of interest for placement, in the region of interest, a dead space, which is free of the biological material and is in fluid communication with a portion of a container containing said biological material. In this connection in some embodiments the system of the present invention may further include a mechanism for positioning the container of the biological material with respect to the optical system, such that the illumination and detection paths intersect with the dead space of the container and traverses a predetermined optical path (length) through the dead space.

In an embodiment of the present invention, the system of the invention and/or its controller (control system) may be configured and operable for carrying out various operations of the method described above and more also described more specifically below.

According to the present invention, the method is used in detection of microorganisms in a biological material. The method according to the invention further includes:

(i) applying non-invasive in-situ optical measurements to a region of interest being a dead space free of a biological material and in a fluid communication with a portion of a container containing the biological material. The optical measurements include illuminating the region of interest with light of a predetermined substantially narrow spectrum including two or more predetermined wavelengths and measuring transmission of said two or more wavelengths through the dead space;

(ii) analyzing measured data of said transmission and generating data indicative of a concentration of the at least one metabolic gas in the dead space which is in fluid communication with the biological material; and

(iii) processing the gas concentration data based on an equilibrium condition between a rate of generation or consumption of the at least one metabolic gas by said microorganisms and a rate of flow of said at least one metabolic gas into and out of the container, thereby generating data indicative of microorganisms in the biological material.

According to the present invention, it is proposed to provide a container including: a sealable main body for containing a biological materials and being permeable to at least one metabolic gas; and a reservoir being configured for fluid communication with the sealed main body, and having at least a portion thereof at least partially transparent to one or more wavelengths corresponding to at least one absorption peak of the at least one metabolic gas.

According to the present invention, it is propoed to provide a reservoir for use in inspection of biological material contained in a sealed platelets storage container permeable to at least one metabolic gas. The reservoir is connectable to the container, so as to be in fluid communication with a dead space in the container (above a portion thereof where the biological material is contained) while the container is maintained sealed with respect to the biological material and contaminates/microorganisms. Also the reservoir has at least a portion thereof which is at least partially transparent to one or more wavelengths corresponding to at least one absorption peak of at least one metabolic gas.

The present invention thus provides an effective and simple technique for accurate in-situ real time non-invasive monitoring of a biological material for detection microorganisms therein. Additional embodiments of the present invention are described in more details in the detailed description below.

In order to better understand the subject matter that is disclosed herein and to exemplify how it may be carried out in practice, embodiments will now be described, by way of example only, with reference to the accompanying drawings, in which:

Fig. 1A is a block diagram schematically illustration a system for detecting microorganisms in biological material according to some embodiments of the present invention;

Fig. 1B is a graph exemplifying absorption cross section of metabolic carbon dioxide (CO2) gas at ambient conditions;

Figs. 1C and 1D respectively illustrate two examples of the spectrum of light beams of first and second wavelengths suitable for use in the invention, where the first and second wavelengths are respectively superposed to overlap/cover an absorption peak (absorption line) and absorption valley (transmission line) of the CO2 absorption spectrum in the mid-IR wavelengths band near 4.3 microns;

Figs. 1E and 1F respectively show logarithmic scale graphs illustrating the transmittance of CO2 for the first and second type wavelengths of Figs. 1C and 1D, and a ration of the CO2 transmission in these wavelengths;

Figs. 2A and 2B exemplify a system for detecting microorganisms in biological material according to some embodiments of the present invention, utilizing a separate closed gas chamber connected to a container of biological material;

Fig. 3 shows the experimental results for a measured plot of %CO2 vs. bacterial concentration;

Fig. 4 shows simulated absorption versus wavelength and CO2 concentrations of the beam of the QCL in the IR spectral range 2355 cm-1 -2410 cm-1 ;

Figs. 5A and 5B show experimental set ups of the system of the present invention used for detection of metabolic CO2 inside a platelets product in a plastic bag utilizing direct measurement through the bag containing the platelets (Fig. 5A) and utilizing a separate gas chamber connected to the platelets container (Fig. 5B); and

Figs. 6A to 6D show experimental results for a fermentation process/run of recombinant protein production utilizing Escherichia coli E. Coli fermentation.

The principles and operation of in-situ real-time and non-invasive detection of microorganisms in biological materials according to the present invention may be better understood with reference to the drawings and the accompanying description.

The invention is based on measuring the absorption of illuminating light (typically in the infrared spectrum) transmitted through a gaseous atmosphere in fluid communication with the biological material, e.g. in a portion of a storage container above the biological material. Living microorganisms produce metabolic gases such as carbon dioxide (CO2) during respiration. By means of infrared absorption, the concentration of metabolic gases can be measured inside the storage container.

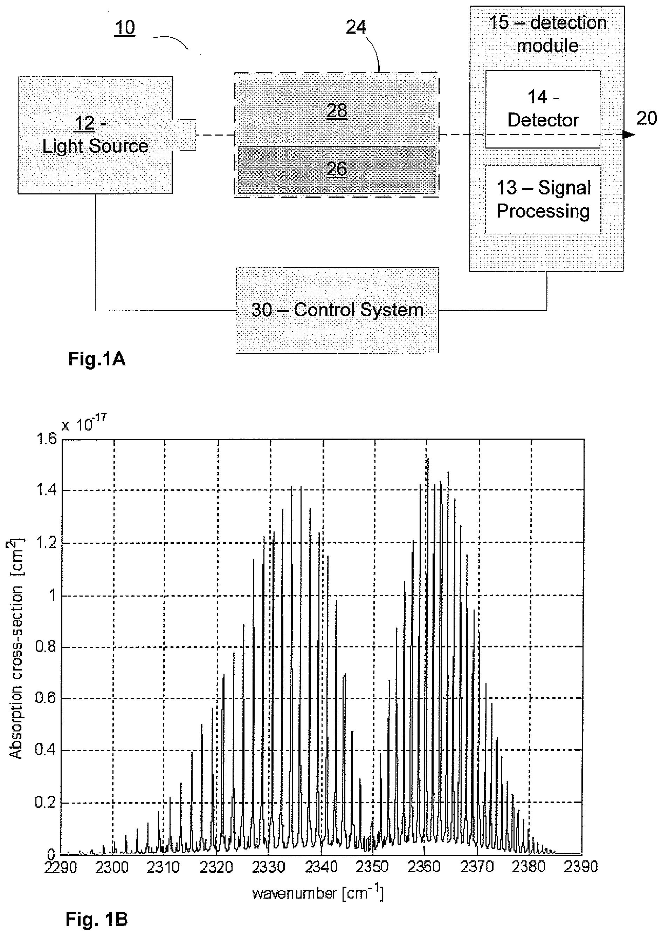

Reference is made to Fig. 1A illustrating schematically in a block diagram a system 10 according to some embodiments of the present invention for detection of microorganisms in a biological material. The system 10 includes an optical system which includes a tunable broadband IR light source 12 and a detection module 15. The tunable broadband IR light source 12 is configured and operable for emitting light in a predetermined substantially narrow spectrum. The tunable broadband IR light source 12 is controllably operated for emitting light in at least a first and a second predetermined wavelengths, wherein the first wavelength corresponds to an absorption peak of at least one metabolic gas to be detected, and the second predetermined wavelength is in a spectral region outside the absorption peak of the at least one metabolic gas. The detection module 15 includes a detector 14 sensitive in the IR wavelength regime. The light source and the detection module are arranged to form respectively illumination and detection paths, e.g. being in optical communication with one another, intersecting a region of interest. The detection module is configured and operable for detecting light in the first and second wavelengths, such as light passing through the region of interest located in between the light source 12 and the detector 14, and generating intensity data/signals indicative of the intensity of detected light in the first and second wavelengths. This data is therefore indicative of the transmittance of the region of interest to the at least said first and second wavelengths.

Further provided in the system **10** is a control system **30** (e.g. controller), which is connectable to the optical system, i.e. to the light source **12** and to the detection module **15.** The controller **30** is configured and operable for operating the light source **12** to emit light in the selected at least first and second wavelengths, and for receiving and analyzing measured/detected data/signals from the detection module and generating data indicative of a concentration of the metabolic gas in the region of interest.

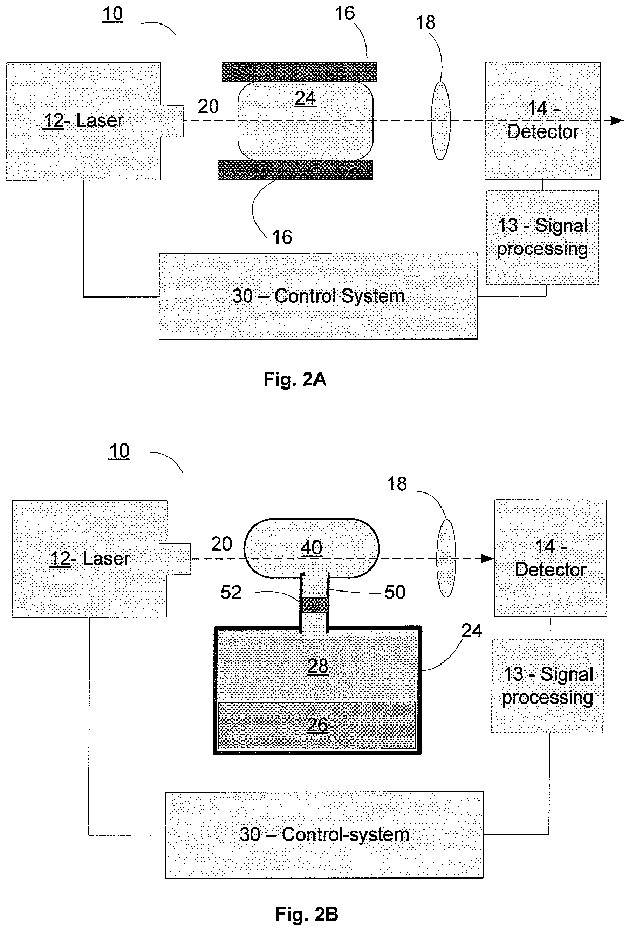

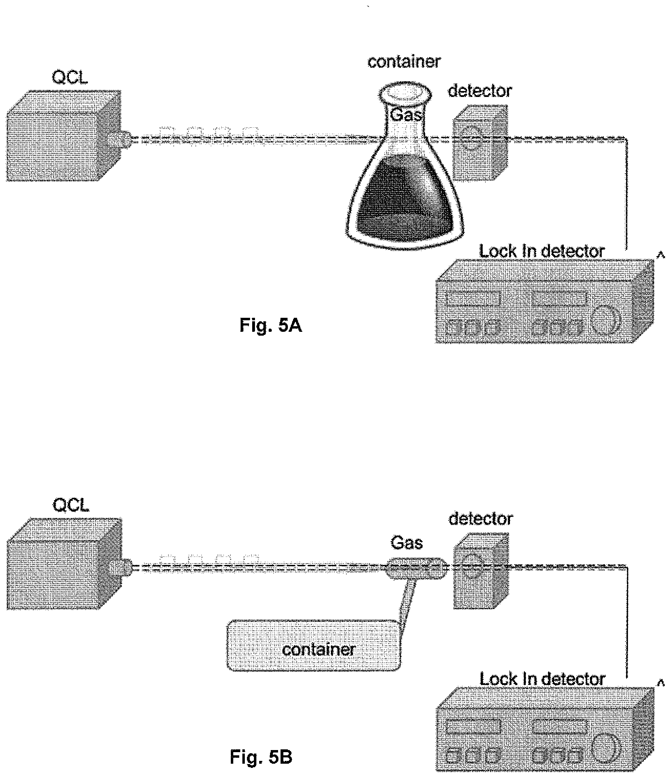

The light source **12** and the detector **14** are arranged in space-apart relationship defining the region of interest therebetween for spectroscopic measurements. To this end, light source **12** and the detector **14** are arranged such that a suitable container **24** of a biological material **26** and/or more specifically a dead space **28** associated with and being in fluid communication with such container **24,** can be placed. The biological material is that in which microorganisms should be detected by optical/spectroscopic measurements performed by the system **10** of the present invention. As indicated above, the *dead space* **28** of the container is actually any space being in fluid communication with the atmosphere in the container above biological material **26.** This may include any one of the following: the portion **28** of the container **24** above the biological material as illustrated for example in **Fig. 1A,** and/or any suitable gas-chamber such as a reservoir and/or an outlet pipe/tube connected to the container and being in fluid communication with its atmosphere, as will be described below with reference to **Figs. 2A-2B** and **5B.**

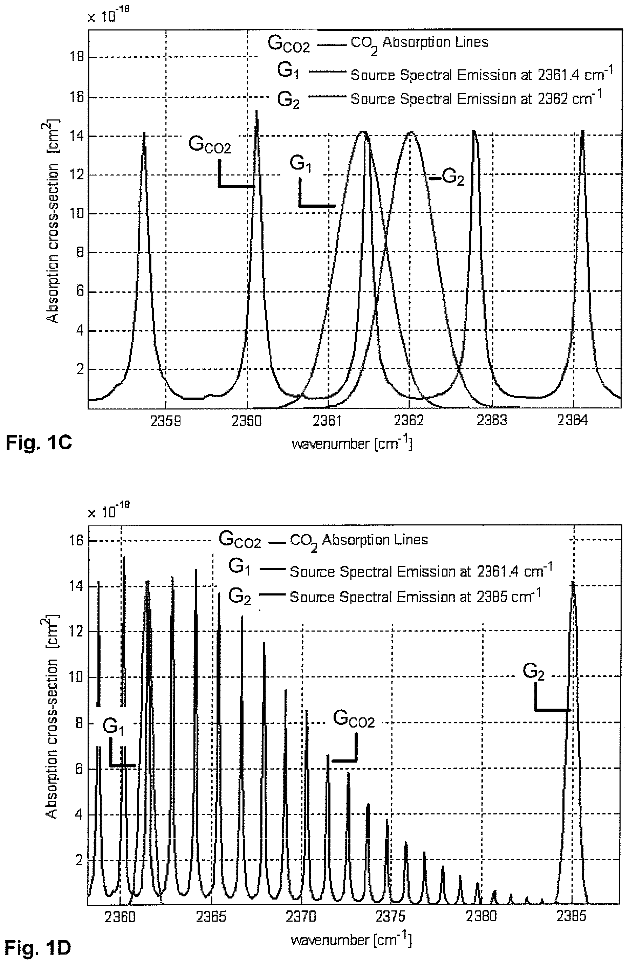

The analysis of the measured data is generally based on the principles of spectroscopy. However, in the present invention, the first and second wavelengths are particularly selected to enable accurate and high-sensitivity measurements of the concentrations of one or more metabolic gases, even in noisy environment, possibly having a wide range of unknown parameters. The first wavelengths is selected to be highly affected by absorbance by the at least one metabolic gas in the region of interest, i.e. to overlap with the absorption line (at times termed herein as "peak"). The second wavelength is on the other hand selected to be less affected by absorbance of the metabolic gas, but nevertheless it is selected to be spectrally close to the first wavelength, such that it provides reference data indicative of absorbance of the first wavelength by other materials in the region of interest. For example the distance between the first and second wavelengths may in some embodiments be about half of distance between two absorption lines of the metabolic gas, or in some embodiments the second wavelength may be located outside the spectral range including intense absorption lines of the metabolic gas. This is exemplified in and described in more details below with reference to **Figs. 1C** and **1D.**

In this regards, it should be noted that the inventors have found that some metabolic gases are associated with spectral regimes of high absorbance in which sharp absorbance peaks exist in the vicinity of sharp transmission peaks (absorbance valleys). On the contrary, the absorption spectra of various materials (e.g. polymer, plastic of which various containers are made as well as vapors such as water vapors), which are in many cases located in the optical path of the measured gaseous media and impede accurate spectroscopic measurements of such metabolic gases, are typically associated with relatively broad and non-volatile spectral profile which does not have sharp peaks. Taking advantage of this finding, some embodiments of the invention utilize the so-selected second wavelength to provide reference to the absorbance of the first wavelength by materials other than the metabolic gas in the vicinity of the region of interest. This can be done because the spectral distance between the first and second wavelengths is selected such, or is sufficiently small, such that the first and second wavelengths exhibit same or nearly similar absorption by various materials possibly located in the optical path.

Thus, the at least two wavelengths are specifically selected such that the one of them is highly absorptive by the metabolic gas to be detected (which concentration is to be measured) and the second is selected to be less absorbed by the metabolic gas but absorbed to similar level (as the first wavelength) by other materials conventionally/typically located in the optical path of measurements. This allows for in-situ non-invasive optical/spectroscopic measurements of the metabolic gas concentration with high accuracy and/or high sensitivity while not reducing the effects of un-known materials/condition in the vicinity and/or in the region of interest of the measurement.

The system and method can be adapted for conducting optical measurements in more than two wavelengths; e.g. with one or more wavelengths of the first type - highly absorbed by the metabolic gas, and/or with one or more wavelengths of the second type - less/negligibly absorbed by the metabolic gas. More specifically a coherent broadly tunable light source (e.g. Mid-IR light source) is used to emit light at first type wavelength overlapping with absorption line(s) of the metabolic gas and having a spectral width wider than that of the absorption line. In the description below the term overlapping is used also to denote that the spectral width of the light source exceeds the spectral width of metabolic gas ($CO_2$) lines. This ensures robustness and high repeatability of the measurements. More precisely, the spectral width of the light source is preferably more than the spectral width of the absorption line, but less than the distance between two adjacent spectral absorption lines. Thus, during a wavelength scan of the laser/light-source, the scanning accuracy with spectral resolution comparable or higher than the spectral width of the light source in order to enable to tune the light source to overlap/cover the absorption line. The use of light source with spectral width exceeding the spectral width of absorption lines provides that spectral resolution of tuning needs not to be higher than the width of the gas absorption line, which may be very small (e.g. wave-number below 0.05 cm$^{-1}$) and which spectral resolution of tuning is hard to achieve. Also, the spectral width of the absorption line is a function of gas pressure and temperature. Hence utilizing a light source having spectral width exceeding the width of absorption line, decreases the sensitivity of the variations of the spectral width of the absorption lines thus improving the robustness of the measurements under varying conditions (temperatures and pressures). Also the condition that the spectral width of the light source is less than the distance between two adjacent spectral absorption lines ensures high contrast and/or resolution when measuring at the second type wavelength, away from the maximum absorption.

In fact, the light source **12** may be a broad-band tunable light source with sufficiently narrow spectral peak. The control system **30** is in communication (by wires or wireless signal communication) with the light source **12** and configured for swiping the wavelength emitted by the light source **12** (continuously or discretely) over a certain wavelength range including several absorption peaks and/or valleys of absorption in the absorption spectra of the metabolic gas to be detected. The control system is also in communication (by wires or wireless signal communication) with the detection module (e.g. for operating it) for receiving measured data indicative of transmission of the several wavelengths in this spectral ranges such that one or more of the wavelengths correspond to the first type wavelength and one or more of them correspond to the second type wavelength. This procedure, in which data on transmission of more than two such wavelengths is acquired, is used in some cases, to further improve the accuracy and sensitivity of the system and method of the present invention event in very noisy scenarios.

It should be understood, although not specifically illustrated, that the control system is typically a computer system including inter alia digital or analog input and output ports, memory, data processor, and may be implemented as hardware and/or software modules. Such a computer system may be at least partially integral with the detection module.

The light source **12** may be configured for emitting substantially monochromatic light, which narrow spectral width is in the order of the width of certain spectral peaks/features in the absorbance profile of the metabolic gas to be detected. For example, in some cases the light source **12** is a tunable IR light source/laser. In particular the light source may be selected to be tunable within a certain wavelength band in the mid-IR regime (the term *mid-IR* is used herein to designate wavelengths of light in the spectral range of 3 to 30 microns. In some particular embodiments of the system **10** of the present invention the tunable quantum cascade laser (QCL) is used as the light source **12,** since it provides wide wavelength tunability and sufficiently narrow spectral width (sufficiently monochromatic light emission). Alternatively or additionally, the light source **12** can also be a broadband source equipped with suitable narrow-band spectral filters in the mid-IR regime. The use of a tunable light source instead of the fixed wavelength source allows for determining the metabolic gas concentration within the container without using any etalon.

The detection module **15** may include an IR detector **14** whose output is connected to an electronic signal processor/amplifier **13**. In some embodiments, the electronic signal processor **13** includes or is constituted by a lock-in amplifier.

In some cases, the at least one first wavelength is selected to be in a spectral regime of high absorbance of carbon dioxide (being the probed metabolic gas) such that first wavelength overlaps an absorption peak/line of carbon dioxide in this regime. For example the at least one first wavelength may be in the wavelength band in the vicinity of 4.3 microns which corresponds to spectral regime of high absorbance by $CO_2$. Indeed, in some cases also the second wavelength is selected close to the first wavelength and in the similar regime of high absorbance by the metabolic gas. However the second wavelength is specifically selected/tuned to fall outside of an absorption line (e.g. in an absorption valley) of the carbon-dioxide/metabolic gas such that it only provides a reference to the absorbance by other material in this regime.

In this connection, reference is now made to **Figs. 1B, 1C and 1D,** in which **Fig. 1B** shows a graph of the absorption cross section of $CO_2$ at ambient conditions (namely pressure of 1 atmosphere and temperature of 25°C) as a function of wave number (taken from HITRAN database), and **Figs. 1C** and **1D** show in more details a part of this absorption cross section of $CO_2$ superposed with two examples of spectral emission profile of the light source for the first and second wavelengths produced by a QCL light source. These profiles correspond to substantially monochromatic light beams of first and second narrow wavelength spectra. In the example of **Fig. 1C** the emission profile of the light source for the first and second wavelengths respectively, overlap/cover an absorption peak (absorption line) of the $CO_2$ and overlap/cover an absorption valley (transmission line) of $CO_2$ in the mid-IR spectrum near 4.3 microns. In **Fig. 1D,** as in **Fig. 1C** the emission profile of the first wavelength, overlaps an absorption line of the metabolic gas, while the second wavelength is located outside a spectral range of intense absorption lines of the metabolic gas.

More specifically **Fig. 1B** shows the features of the CO2 absorption spectrum in wave-number range of near about 2300 to 2380 $cm^{-1}$ (i.e. for mid-IR wavelength range of about 4.2 to 4.35 microns).

**Fig. 1C** shows in more details the absorption spectrum (graph $G_{CO2}$) of CO2 in the wave-number range of about 2359 to 2364 $cm^{-1}$ (i.e. for wavelengths ranging between 4.230 to 4.239 microns) and illustrates the typical width of CO2 absorption lines of about 0.07 $cm^{-1}$ (namely in the order of about 0.15 nm in that wavelength range).

**Figs. 1C** and **1D** present narrow spectral profile (graphs $G_1$ in these figures) of a first light beam (first type wavelength) produced by a tunable QCL light source **12** used in some embodiments of the present invention. The typical spectral width of the QCL emission is of *full-width-half-maximum* (FWHM) of about FWHM = 0.7 $cm^{-1}$ (i.e. about 1.25 nm), and the figures show said spectrum of the light-beam being tuned to a central wave-number of 2361,4 $cm^{-1}$ (wavelength of about 4234,8 nm microns) corresponding to the maximum of one of the absorption lines of the $CO_2$ gas.

In the example of Fig. 1C the second wavelenth presented by graph $G_1$ was tuned to central wavenumber of 2362 $cm^{-1}$ (wavelength of about 4233,7 nm) which is in the middle between the two absorption lines of the metabolic gas. Thus, in this case the $CO_2$ absorption at the second wavenumber will be much lower, than at the first one.

In the example of Fig. 1D the second wavelength presented by graph $G_2$ was tuned to central wavenumber of 2385 $cm^{-1}$ (4192,9 nm). Therefore in this example the second wavenumber is outside the spectral range/band of high absorbance by the metabolic gas (e.g. outside the so called 4,3 micron absorbance band of $CO_2$ at which main $CO_2$ absorption lines are present). Accordingly the $CO_2$ absorption at 2385 $cm^{-1}$ wavenumber is much lower, than its absorbance at both at the 2361,4 $cm^{-1}$ and 2362 $cm^{-1}$ wave-numbers.

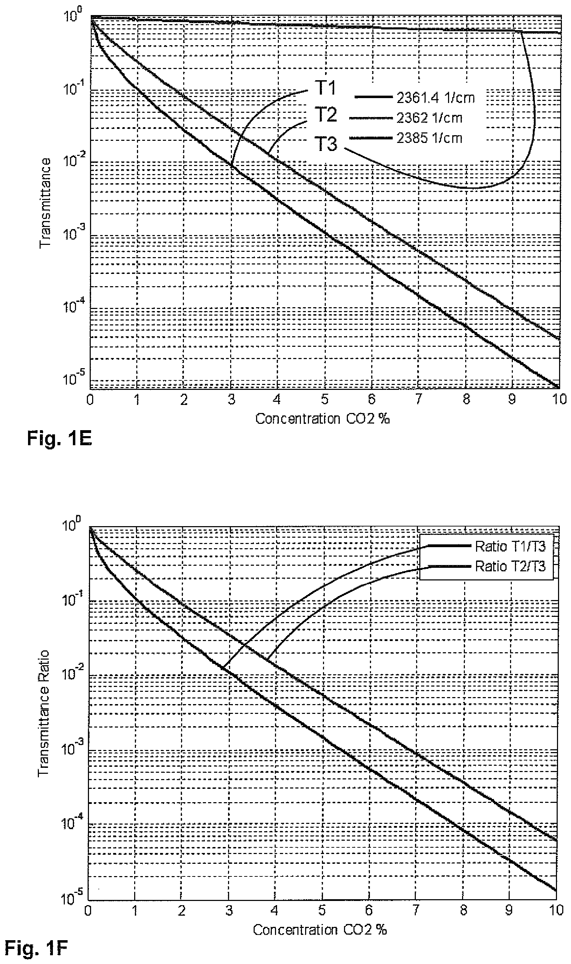

The transmittance of $CO_2$ for the above exemplified wave-numbers of the first and second type wavelengths is illustrated in logarithmic scale in Fig. 1E for various concentrations of the metabolic gas $CO_2$. The transmittance was simulated for a container with transparent walls filled with mixed $N_2$ and $CO_2$ gases at different CO2 concentrations, at normal pressure, normal/room temperature of 300 K, and with optical absorption path of 8 cm. The transmission of the first type wavelength (graph $G_1$ in Figs. 1C and 1D corresponding to wave-number 2361.4 cm-1) is illustrated in graph T1. The transmission of the second type wavelengths (graphs $G_2$ in Figs. 1C and 1D) corresponding to wave-numbers 2362 $cm^{-1}$ and 2385 $cm^{-1}$ are illustrated in graphs T2 and T3 respectively. As shown from these graphs, the absorbance of the first wavenumber in the $CO_2$ (graph T1) is substantially higher than the absorbance of the second type wavenumbers (graphs T2 andT3).

Fig. 1F shows in self explanatory manner the transmittance ratios T1/T3 and T2/T3. These ratios are monotonically decreasing functions in both cases. Accordingly in some embodiments of the present invention it may be sufficient to measure the $CO_2$ absorbance to obtain the value of one such ratio which is sufficient to for unambiguous determination of the $CO_2$ concentration in the container. However, in some embodiments of the present invention the reliability of the measurements are further improved by utilizing more than two wavelengths (e.g. to obtain more than one such ratio). That is especially relevant for high concentrations of $CO_2$ gas (exceeding few per cent level) where absorption at certain wavelengths could be very strong and measured signal very weak.

To this end, the controller operates/tunes the light source to emit light in the first substantially monochromatic wavelength(s) (e.g. as illustrated in graph **G1**) corresponding and overlapping/covering the high absorbance peaks/lines of the metabolic gas such as those of CO2 illustrated in the figure, thereby providing accurate measurement of the CO2 absorbance. Additionally, as noted above, the controller operates/tunes the light source to emit light in second substantially monochromatic wavelength(s) (e.g. as illustrated in graphs **G2** of **Figs. 1C** and **1D**) corresponding to regions/valleys of low absorbance of the metabolic gas (e.g. transmission lines between the absorption lines which spacing is in the order of about 1 $cm^{-1}$ to 1.5 $cm^{-1}$ - about 1.8nm-2.5nm in wavelength, and/or regions outside the high absorbance band of the gas). These second monochromatic wavelength(s), while being narrow and tuned so as to substantially not overlap with the absorption lines are provided for reference to the absorption of materials in the optical path other than the probed metabolic gas. Further, these second wavelengths are in the general neighborhood/band of the first wavelengths such that they provide reference data being indicative, with good accuracy, of the absorption of the first optical wavelength(s) in the optical path, in case the probed metabolic gas was absent there. In other words, the second predetermined wavelength is selected such that measured data of the transmission thereof provides reference indicative to the optical absorbance of the first wavelength by materials other than the metabolic gas.

For example, in some cases a spectral distance between a "first type" wavelength of the emitted light and a "second type" wavelength serving for the reference measurement (to which the metabolic gas is substantially transmitting) is in the order of 0.5 $cm^{-1}$-0.75 $cm^{-1}$ that corresponds to the half of distance between two absorption lines of CO2 gas. Yet, in other case the separation distance can be of order 30 $cm^{-1}$ for the second absorption line to be outside the whole spectral range with intense absorption lines. In that case this wavelength could be used as reference wavelength transmittance of light beam at this wavelength does not depends on the concentration of CO2, but rather reflects absorptions of the light beam on other optical components such as container walls transmittance. Hence, the spectral distance between the first and second wavelengths is substantially small such that the first and second wavelengths are characterized by same or similar transmission through predetermined/conventional materials used for conventional containers of biological material.

The use of such first type wavelength(s) for the measurement of absorbance by the metabolic gas and the second type wavelength(s) close thereto for reference measurements allow to detect small/minute changes in the metabolic gas concentration with high sensitivity and accuracy. For example changes of 10 ppm or 0.001% in the gas concentration can be detected even in relatively noisy environments in which various other materials such as those of the walls of the storage-bag and/or fermentation container and/or vapors (e.g. water vapors) are located in the region of interest. The effects of the later can be discarded with accuracy based on the reference measurements of the second wavelengths being close to the first wavelengths.

As a result of the above, in some cases the technique of the present invention is employed to measure metabolic gas concentrations in conventional containers such as conventional storage bags/vials for platelets. This is achieved by taking advantage of the above described technique utilizing the "first type" and "second type" (measurement and reference wavelengths) in the spectroscopic measurements. Thus, the detection of various microorganisms *in-situ* in such conventional and generally arbitrary containers is made possible although the materials of the container is not *a-priory* known, and although the container's materials may include materials such as polymers and/or other materials, which may be relatively opaque to the IR wavelengths used by the system of the invention (e.g. opaque to wavelengths in the mid-IR).

As noted above in some embodiments the detection module **15** includes an electronic signal processor/lock-in amplifier **13** that receives the signal from the IR detector **14**. Use of the lock-in amplifier enables to even further improve the signal to noise ratio (SNR) provided by the system thus further improving the sensitivity and accuracy of the measurements relating to the concentration(s) of metabolic gases and consequently detection of microorganisms. To this end, in such embodiments the control system **30** is adapted for operating the tunable broadband IR light source **12** for applying time modulation to intensity of light emitted in one or more (e.g. in each) of the at least two (first and second) wavelengths, and also operating the lock-in amplifier **13** to determine/measure the detected intensity(ies) of the emitted light with high accuracy based on that modulation. Accordingly, transmittance of the region of interest to the first and second wavelengths (e.g. to all wavelengths used in the measurement) can be determined with high accuracy based on the intensity modulation, while noise is mostly discarded as it is generally not modulated in the same way. It should be noted that the configuration and operation of various lock-in amplifiers are generally known in the art of signal processing and are therefore not specifically described herein. A person versed in this art would readily appreciate the various possible configurations of such lock-in amplifier with appropriate modulation to the emitted illumination to be used in the system of the invention.

The system **10** is configured and operable for utilizing its ability to accurately detect small changes in the metabolic gas concentration, for operation *in-situ* and/or in real time to non-invasively detect microorganisms in the containers/bags permeable to the metabolic gas and/or in association with one or more openings (inlets/outlets) through which in/out flow of gases may occur. Specifically, in some embodiments, after analyzing the concentrations of the metabolic gas in the dead space **28**, the controller **30** is adapted for further processing the concentration of metabolic gas to detect microorganisms in the biological material **26**.

In some cases, the detection is merely qualitative to identify whether significant levels/amounts of such microorganisms exist in the biological material **26**. In other cases, the detection is qualitative and is aimed at estimating the levels (e.g. amounts/concentrations) of the microorganisms in the biological material **26**.

The system is adapted/configured for operating in closed containers, sealed and non-permeable to the probed metabolic gas(es). In such cases, the concentration of the metabolic gas in the container is a function of the time the container is sealed and the amount of microorganisms therein during that time. Specifically, in sealed container the concentration of CO2 is increasing function with time as long as there is biological activity inside of container responsible for CO2 emission. Thus, the concentration of living cells in that case depends on the growth history of the cells from the beginning of incubation. The correspondence between the number of cells and measured CO2 concentration could be find out in that case by computing rate of changes of CO2 concentration and correlating it with respiration rate of a single cells and the number of cells.

Alternatively or additionally, the container is not sealed with respect to the metabolic gas. For example, in cases of monitoring a biological material stored in bags or storage vials for blood components, which are permeable to the metabolic gas, and/or in cases of monitoring a biological material in fermentation containers associated with gas inlet(s) and/or outlet. Advantageously, the present invention allows for detecting microorganisms also in such containers *in-situ* and in real time without taking and sealing a sample of biological material from the containers and without incubating the sample (i.e. non-invasive detection). This is performed by taking advantage of the high sensitivity and accuracy of the technique of the invention for detecting small changes in the metabolic gas concentration.

To this end, the controller **30** system may operate to measure the metabolic gas concentration in a dead space associated with the container of biological material, while the dead space is non sealed to the metabolic gas, and to utilize the measured concentration of the metabolic gas to detect the microorganisms qualitatively and/or qualitatively based on an equilibrium condition (e.g. balance/difference) between a rate of escape/flow of the metabolic gas from/into the container and a rate of generation or consumption of the metabolic gas by the microorganisms.

In cases the container is permeable to the metabolic gas, processing may be performed by computing/estimating the microorganisms level in the biological martial **26** based on the measured concentration of the metabolic gas diffusion of that gas through walls of the container **24.** In this case, the container may be a conventional storage container for platelets or other blood components.

The system **10** of the invention allows real-time in- situ detection of microorganisms in biological material **26** contained in fermentation container **24** (e.g. in a container of a fermentation system). To this end, the controller **30** may be configured and operable to determine the level of microorganisms based on the equilibrium condition (e.g. balance/difference) between the rate of escape/flow of the metabolic gas from/into the container **24** through an outlet thereof, and a rate of generation or consumption of the metabolic gas by the microorganisms. For example that balance may be determined based on a difference between concentrations of the metabolic gas in a gas inlet to the container **24** (e.g. the concentration in the external atmosphere) and a concentration of that metabolic gas in the atmosphere in the dead space **28** of the container, which may be a dead space of the container itself (e.g. above the biological material **26**) or a dead space in fluid communication therewith, for example located at a gas outlet of the container.

The difference in the metabolic gas concentrations corresponds to the amount of microorganisms in the container. More particularly, this is the difference that may be computed from the difference in the amount of metabolic gas flowing in and out of the container (e.g. computed by the controller **30** as the difference between the products of the flow rates in the gas inlet and outlets multiplied by the metabolic gas concentration thereat respectively).

The controller **30** may utilize predetermined data of the concentration of the probed metabolic gas in the gas inlet (e.g. in the external atmosphere of the container. Also the spectroscopic/optical measurements may be applied to a region of interest associated with the cavity/dead-space of the container itself and/or at a gas outlet from the container. The light source **12** and the detector **14** may be optically coupled to one or more optical windows exposing the dead-space to be inspected (e.g. optical windows coupled to the fermentation container and/or to gas outlet).

In an embidiment system **10** is specifically configured for real time and continuous/periodical monitoring of fermentation processes. In such embodiments the controller **30** may be adapted to apply continuous/periodic detection of microorganisms and/or their level in the fermentation container. In this connection, the controller **30** may be adapted to repeatedly operate the optical system in the manner described above to obtain, in real time, and/or repeatedly within predetermined time intervals, data indicative of the metabolic gas concentration in the container and/or in its outlet. Repeatedly/continuously obtaining such gas concentration during a period of time provides indication to the amount of microorganisms in the container as a function of time and/or indication to the change/rate of change in this amount. The controller may be adapted to process the data indicative of the amount of microorganisms, or the change thereof, as function of time for monitoring and/or controlling the fermentation process occurring in the container. For example, reference data/model relating to the fermentation process in a container may be utilized by the controller to determine actions/operations to be carried out for controlling the fermentation (e.g. stopping the fermentation and/or changing some of the fermentation conditions such as temperature and/or other conditions). This reference data may for example be stored in the form of a lookup table (LUT) and/or a set of one or more functions/models relating to the amount of micro-organisms and/or rate of change in their amount with certain actions to be carried out and/or certain fermentation conditions to be applied/maintained.

The fermentation process monitoring/controlling can be realized using the a model (e.g. mathematical-model/formula and/or data) relating the amount of biomass in the fermentor with measured concentration of CO2. The model may be pre-determined and predefined in advance and loaded to memory and/or other storage device of the controller **30** in the form of data/LUT and/or as a set of instructions soft/hard coded.

Such a model relating the amount of biomass in fermentor with the concentration of $CO_2$ may be obtained/determined in advance by utilizing various techniques. For example, the amount of biomass may be measured by optical density techniques (OD) and/or viable counts and/or other run parameters such as pH, RPM, TEMP, and the total volume (TV) of the metabolic gas $CO_2$ emitted/consumed by the microorganisms from the beginning of the run (seeding time).The model may be a mathematical model based for example on multivariable robust regression analysis. Verification of the mathematical model may be performed by number of fermentation processes performed under same conditions, which are optimal for the high product yield in the batch fermentor.

A RUN protocol, such as executable instructions and/or LUT, is then used by the controller **30** for estimating, the amount of biomass in the fermentor based on the model's parameters and the real-time measured concentration of CO2 gas and possibly other run parameters. The RUN protocol is based on estimated values of the biomass and may include data indicative of different aspects/actions to be taken during the monitoring of the fermentation process (e.g.

conditions for adding nutrients for cell in fermentation, determining optimal inducing time (for recombinant protein production) and harvesting time, controlling pH level and other run aspects.

As noted above, in cases of biological containers which are not-sealed to the metabolic gas (e.g. permeable containers or containers of fermentation), the bacterial growth will be reflected in real time in changes in the metabolic gas concentration. For example, in case the bacteria are no longer alive, the carbon dioxide concentration will be substantially equal to equilibrate with that in the air outside the storage tank.

The concentration of metabolic gases inside a gas permeable container is determined by equilibrium conditions between release and rate of diffusion of metabolic gases through the walls of the container. It should be noted that in the following description containers permeable to the metabolic gas(es) are considered as an example of not-sealed containers with respect to the metabolic gas(es). Also, in the description below such permeability and diffusion of the gas from and into the container is exemplified as relating to the permeability of the container's walls. However, it should be understood that the technique described below is applicable to any other type of containers no sealed with respect to metabolic gases, wherein the flow in and out of the container may be additionally or alternatively through gas inlets and/or outlets of the container. Further, in connection with the diffusion equations used in the description below, it should be noted that in case of non-permeable non-sealed containers these can be substituted by proper equations taking into account other "diffusion" paths such as flow through inlet/outlet pipes and concentration of the metabolic gas therein. In other words, although the examples in the description below mainly refers to permeable platelets bags, it should be understood that the technique can easily be generalized by a person versed in the art for other types of cavities where metabolic gas may be concentrated, including the dead space in the container itself and/or that of the inlet/outlet pipes of a fermentation container for example.

The equation for gas (such as $CO_2$) production and transport through the walls of a permeable container states

$$\frac{\partial N_{CO2}}{\partial t} = -JA + W \tag{1}$$

where $N_{CO2}$ is the number of $CO_2$ gas molecules, $J$ is the diffusion flux from the walls of the container, $A$ is the surface of the walls exposed to the gas exchange and $W$ is the source term that describe total rate of $CO_2$ production inside the container. The diffusion flux is given by

$$J = (n_{in} - n_{out})K \tag{2}$$

where $n_{in}$ is the concentration of $CO_2$ inside the container, $n_{out}$ is the ambient concentration of $CO_2$ outside the container and $K$ is the membrane permeability coefficient. $W$ for bacteria contaminated product is given by

$$W = N_{bact}r_{bact} + W_0 \tag{3}$$

where $N_{bact}$ is the number of bacteria inside the container and $r_{bact}$ is $CO_2$ emission rate by bacteria in units m3/sec and $W_0$ is the product $CO_2$ emission rate. In equilibrium condition, the total $CO_2$ emission rate is equal to the rate of diffusion through the container walls

$$\frac{\partial N_{CO2}}{\partial t} = 0 \tag{4}$$

Thus the number of bacteria $N_{bact}$ is related to the concentration of CO2 gas $n_{in}$ as

$$N_{bact} = \frac{1}{r_{bact}}\left((n_{in} - n_{out})KA - W_0\right) \tag{5}$$

If the product contains no bacteria, then $N_{bact} = 0$ and initial concentration of CO2 gas inside the container is

$$n_{in0} = n_{out} + W_0/KA \tag{6}$$

Then from Eq. (5) above, a change of CO2 concentration $\Delta n_{CO2} = n_{in} - n_{in0}$ is related to $N_{bact}$ as

$$N_{bact} = \frac{\Delta n_{CO2} KA}{r_{bact}} \qquad (7)$$

Thus, the number of bacteria is proportional to the change of $CO_2$ concentration $\Delta n_{CO2}$, to the container wall permeability coefficient $K$ and to the container wall area $A$, and is inversely proportional to the CO2 emission rate by a single bacteria $r_{bact}$.

The equation (7) was derived under assumption of equilibrium conditions for gas emission and diffusion through the container wall. If container is sealed, then this equation is inapplicable, since no gas exchange can undergo through the container walls. In this case, the concentration of CO2 is gathered by Eq. (1) with the first term in the right side equal to zero:

$$\frac{\partial N_{CO2}}{\partial t} = N_{bact} r_{bact} + W_0 \qquad (8)$$

In that case, $N_{CO2}$ is increasing with time as long as there is biological activity inside the container responsible for CO2 emission.

The equation (7) provides means for detecting biological activity in a product stored in gas permeable container. The procedure is especially simple if the only source for emission of CO2 gas inside the container is bacteria. In that case, $W_0 = 0$ and concentration of CO2 gas inside the container is equal to the ambient concentration: $n_{in0} = n_{out}$ as follows from Eq. (6). In that case, increase of concentration $\Delta n_{CO2}$ above the ambient will indicate on presence of biological activity inside the container.

If $W_0 > 0$, then detection of bacterial contamination can be done by measuring change $\Delta n_{CO2}$ of CO2 concentration relative to some reference concentration of uncontaminated product.

The change of concentration $\Delta n_{CO2}$ can be measured by means of IR absorption of beam of tunable IR light source such as Quantum Cascade Laser directed through the container walls. As described above, the use of the tunable source instead of the fixed wavelength source allows direct measurement of CO2 concentration inside the container regardless of container material and without use of etalon container.

The following are some examples of the technique of the present invention for detection of metabolic gas(es) concentration, assume that the container walls are at least partially transparent at the mid-IR frequency range where strong absorption of CO2 occurs (around 2260cm$^{-1}$-2390cm$^{-1}$), and that the path for optical beam in the gaseous atmosphere inside the container is provided.

The dependence of %CO2 level on the increase of bacterial contamination was studied experimentally. Staphylococcus epidermidis obtained from the American Type Culture Collection (ATCC) were used to contaminate a bag of platelets that were collected from a single donorby apheresis. The bacterially inoculated apheresis platelets were agitated at 22°C and measurements were performed using QCL spectroscopy. The platelet container was measured before and during bacterial contamination. Samples were taken from the contaminated platelet bag and a standard culture plate count was used for determining bacterial concentration [colony forming unit (CFU)/mL] in the platelet medium.

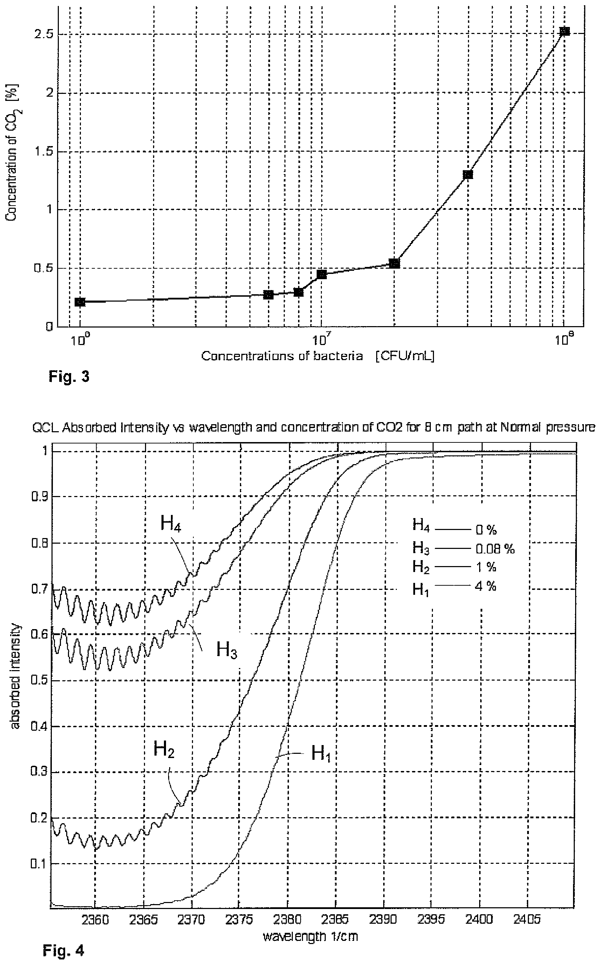

Referring now to the drawings, **Fig. 3** is a plot of %CO2 vs. bacterial concentration. The bacterial concentration that was measured at the point where %CO2 started to rise was between 1*10$^6$ CFU/mL to 6*10$^6$ CFU/mL. The Y-axis shows %CO2 level and the X-axis shows bacterial concentration measured using standard titration analysis.

Turning back to **Fig. 1A,** system **10** of the present invention may be used for measuring the concentration of carbon dioxide in the dead space **28** above the platelets **26** in a gas-permeable bag **24** that has been removed temporarily from storage and agitation for the purpose of measuring the concentration of carbon dioxide in dead space **28**. The tunable infrared laser **12** (for example a QCL) and an infrared detector **14** are positioned so that the light beam **20** from laser **12** is aimed at detector **14**. In this example, the light beam **20** is focused on detector **14** by a calcium fluoride lens **18**. Bag **24** is positioned between laser **12** and detector **14** so that light beam **20** traverses dead space **28**. Controller **30** tunes laser **12** to emit light beam **20** at selected wavelengths in the vicinity of 4.3 microns at a pulse repetition rate of 5 KHz, receive the corresponding response signals from detector **14,** and analyze those signals to estimate the concentration of carbon dioxide in dead space **28**. As noted above, the signal reception and analysis portion of the controller **30** may be implemented by a lock-in amplifier that locks onto the 5 KHz signal from detector **14** and displays the amplitude and phase of that signal. For an accurate measurement of the concentration of carbon dioxide in dead space **28** the path length of light beam **20** across the interior of bag **24** is preferably at least several centimeters.

In practice, a sufficiently long optical path through bag **24** may not be available, and/or the walls of bag **24** may not be sufficiently transparent at the relevant wavelengths to allow an accurate measurement of the concentration of carbon dioxide in dead space **28**. In this connection, reference is made to **Figs. 2A and 2B** illustrating two modified configurations of system **10** that deal with these problems. System **10** is configured generally similar to that of Figs. 1A.

As shown in **Fig. 2A,** the bag **24** may be held in place by two vertical walls **16.** In distinction to the system of Fig. 1A, in **Fig. 2B** a separate closed (e.g. cylindrical) gas chamber (pipe/reservoir) **40** is used and is connected to the bag **24** using a connecting tube **50** which is by its one end connected by fusion with a heating instrument to the bag, as is routinely done to platelet bags in blood banks, for various reasons of their own. Such procedures are performed routinely without damaging the platelet bags or introducing contamination. The other end of the connecting tube **50** is connected to the chamber **40** that is at least partially transparent in at least a portion thereof to the relevant wavelengths and that is sufficiently rigid and long enough to provide a predetermined fixed optical path (e.g. of several centimeters) for light beam **20**. In this specific but not limiting example, a filter **52** is used in tube **50,** that is permeable to gases but not to liquids, and thus keeps liquids from bag **24** out of reservoir **40** but allows the gaseous contents of reservoir **40** to equilibrate with the gaseous contents of dead space **28** in the bag **24** so that the concentration of carbon dioxide in reservoir **40** is substantially identical to the concentration of carbon dioxide in dead space **28**. The equilibration of the concentration of carbon dioxide between reservoir **40** and dead space **28** occurs sufficiently fast such that no special steps are needed to hasten this equilibration. Effectively, the interior of reservoir **40** is an extension of dead space **28**. Such connection to the separate gas chamber may be used in case the required optical path (for detection of the specific metabolic gas) inside the container is unavailable and/or in case the container's walls totally block the IR radiation.

In a general case, irrespectively of whether the container is sealed or permeable with respect to the metabolic gases to be detected, the following technique may be carried out by the controller **30** for accurate, in-situ real time determination of the metabolic gas(es) concentration. IR transmission through the dead space in two or more wavelengths (comprising the above described first and second wavelengths) is measured as described above. The measured data is processed by the controller **30** for example by utilizing an absorption model of the at least one metabolic gas. To this end, a best fit between intensity values obtained from the absorption model and the measured intensity values is performed to thereby determine the concentration of the at least one metabolic gas.

The following is a not limiting example of the mathematical description of the measurement procedure of metabolic gas concentration inside a container using tunable IR light source.

The transmitted laser light intensity $I(\lambda_0)$ measured on the detector is given by at the laser central wavelength $\lambda_0$ is given by

$$I(\lambda_0) = \eta I_0 \int_{\lambda_{min}}^{\lambda_{max}} f(\lambda - \lambda_0) e^{-\alpha_\lambda(cl+c_0l_0)} d\lambda \qquad (9)$$

where $I_0$ is the laser intensity, $\eta$ is the total intensity loss that are not related to optical gas absorption, $\alpha_\lambda$ is the absorption coefficient (in cm$^{-1}$) at the given wavelength of the light $\lambda$, $c$ is the probed gas concentration (by volume) inside the container, $c_0$ is the concentration of the probed gas outside the container in the atmosphere, $l$ is the pathlength inside the container, $l_0$ is the pathlength outside the container between IR source and detector, $f(\lambda - \lambda_0)$ is the laser spectral distribution function around the central wavelength $\lambda_0$.

The integration limits $\lambda_{min}$ and $\lambda_{max}$ with $\lambda_{min} < \lambda_0 < \lambda_{max}$ are assumed to be such that $f(\lambda)$ is nearly zero outside the integration domain.

The absorption coefficient $\alpha_\lambda$ can be calculated as:

$$\alpha_\lambda = n\sigma(\lambda), \qquad (10)$$

where $n = P/k_B T$ is the concentration of molecules and $\sigma(\lambda)$ is the absorption cross section in cm$^2$.

The signal on the detector is assumed to be proportional to the transmittance intensity. In case of tunable laser the central wavelength can be changed within certain range.

Thus, a model $S(x,\lambda_i)$ for a signal on the detector can be written as

$$S(x, \lambda_i) = b \int_{\lambda_{min}}^{\lambda_{max}} f(\lambda - \lambda_i) e^{-\alpha_\lambda(x+c_0l_0)} d\lambda \qquad (11)$$

where $x = cl$ and $b$ is a constant, $x$ (and therefore $c$) can be found from equation (11) if the measurement is done at two or more wavelengths of light $\lambda_i$. In that case the unknown constant $b$ can be excluded from the set of equations.

The concentration $c$ can be determined from $n$ measured values of the signal $S_i i = 1, ..., n$ at different wavelengths $\lambda_i$ by utilizing nonlinear minimization of the model $S(x,\lambda_i)$ as provided by function $s(x)$ below:

$$s(x) = \sum_{i=1}^{n-1} \left[ \log \left( \frac{S(x,\lambda_i)+\epsilon}{S(x,\lambda_n)} \right) - \log \left( \frac{S_i+\epsilon}{S_n} \right) \right]^2 \qquad (12)$$

where $\epsilon$ is a noise level at the detector. $s(x)$ is essentially least squire norm of the logarithm of the ratio between measured and theoretical signals $S_i$ and $S(x,\lambda_i)$ at wavelength $\lambda_i$ $i$ = 1, ... , $n$ - 1 and the signal at $\lambda_n$. Thus, $\lambda_n$ is used as a reference wavelength for $\lambda_i$ $i$ = 1, ..., $n$ - 1 . The parameter $\epsilon$ insures that function $s(x)$ is not singular if one of $S_i$ = 0. From equation (11) the concentration $c$ can be determined, provided that the optical path length $l$ is known.

The following is a specific example for using the technique of the invention for CO2 absorption simulations and evaluation. The simulations of CO2 absorption within plastic bags were performed using HITRAN database of CO2 line intensities at ambient conditions and are described above with reference to Figs. 1B and 1C.

**Fig. 4** shows simulated absorption versus wavelength and CO2 concentrations of the beam of the QCL calculated using equation (9) in the IR spectral range 2355 cm$^{-1}$-2410 cm$^{-1}$. The graphs $H_1$, $H_2$, $H_3$ and $H_4$ correspond to the absorbed intensities for respectively 4%, 1%, 0.08% and 0% of the CO2 concentration. As shown, the graphs have smooth behavior of the spectra on wavelength and the transmittance increases when light frequency changes from 2360 cm$^{-1}$-2410 cm$^{-1}$

Reference is made to **Figs. 5A and 5B** showing experimental set ups of the system of the present invention for detection of metabolic CO2 inside a platelets product in a plastic bag utilizing respectively direct measurement through the bag containing the platelets (Fig. 5A) and utilizing a separate gas chamber connected to the platelets container (Fig. 5B). As shown in the figures in a self-explanatory manner, the set up utilizes tunable QCL, IR detector (e.g. equipped with a CaF2 plano-convex lens), lock-in amplifier detector and a controller. The QCL operates in the pulse mode with repetition frequency of 5 kHz and pulse width 500 nsec. The tunability range of the QCL includes the measurement range that was from 2361.4 cm$^{-1}$ till 2391cm$^{-1}$. The procedure for determination CO2 concentration within container may thus be as follows: The light beam is transmitted from QCL through a small container connected to the plastic bag container as shown in **Fig. 2B** described above. Signal on IR detector is measured at different wavelengths of light $\lambda_i$ $i$ = 1,...,$n$ in the range from 2361.4 cm$^{-1}$ till 2391 cm$^{-1}$. The concentration c of CO2 gas inside the container is estimated using equation (12) for different $\lambda_i$ using the nonlinear minimization of the function $s(x)$.

As noted above in embodiments of the present invention configured for monitoring and/or controlling fermentation processes, the monitoring and controlling of the fermentation may be based on a model such as formula and/or reference data relating various parameters of the fermentation including metabolic gas concentration with an estimation of the biomass (i.e. it amount) in the fermentation. In some cases a *Linear Regression Model* is used for estimating the biomass during the fermentation process.

In this connection, the regression model may be pre-computed model calculated utilizing statistical regression analysis for modeling the relationship between one or more parameters, also considered as to as predictor $x$ and/or regressor variable(s), and one or more other parameters, also referred to as response variable(s) $y$.

For the case of estimating of biomass in the fermentation, the regressor/predictor variable(s) (generally denoted herein $x_i$) include the concentration $C$ of $CO_2$ measured in the gases emitted from the fermenter, and/or *TV* being the *Total Volume* of CO2 emitted from the beginning of the run (seeding time). The response variable (generally denoted herein y) may include the optical density *OD* of the biological material in the container and / or the viable count *VC* (namely a measure of the count of microorganisms as typically obtained via microscope count). In cases that multiple linear regression model is used the response variables - generally noted $y$ are related to $k$ repressor's, $x_1, x_2, ..., x_k$, according to the following formula:

$$y = \beta_0 + \beta_1 x_1 + \beta_2 x_2 + \cdots \beta_k x_k + \epsilon \qquad (13)$$

where $\beta_i$, $i$ = 0, ..., $k$, are model parameters (e.g. coefficients of the model), $x_i$ are the regressor/predictor variable(s) (e.g. parameters of the fermentation process which are independent from the response variable (OD in this case)), and $\epsilon$ is the random components with supposed mean zero and variance $\sigma^2$.

The regressor/predictor variable(s) $x_i$ may be measured in real-time during the fermentation process (e.g. without sampling/extracting the material from fermentor and probing). For example, the total volume (*TV*) of CO2 gas emitted by microorganisms vs. time may be calculated using the formula:

$$TV(t) = \int_o^t rate \times c(t)dt \quad (14)$$

where *TV*(t) (in liters) is the total volume of CO2 gas emitted by the species since the beginning of the run (from the beginning of the fermentation process), *rate* is the aeration rate (e.g. measured in liters/min, and c(t) is the concentration of CO2 (in volume fraction) measured in emission gases from fermentor.

In this connection, in addition to the CO2 concentration predictor parameter c(t), in some cases other run/predictor parameters/variable(s), may also be measured (e.g. continuously) to provide estimation of the response parameter(s) y with improved accuracy. To this end the generalized formula (13) above may be used as a model defining the relation between the predictor parameters measured/considered $x_i$ and the response parameters *y*. For example the predictor parameters $x_i$ may include:

- *RPM* - agitation rate in fermentor (e.g. provided as an input from a controller controlling the operation of the fermentation system);
- *RATE* -aeration rate measured in liters /min (e.g. measured at the inlet and/or outlets from the fermenter);
- *pH* - acidity level in fermentor (e.g. measured by pH electrode in the fermenter)
- *DO* - dissolved oxygen $dO_2$ (e.g. measured by dissolved oxygen probe in the fermenter)
- *Temp*- temperature in fermentor,

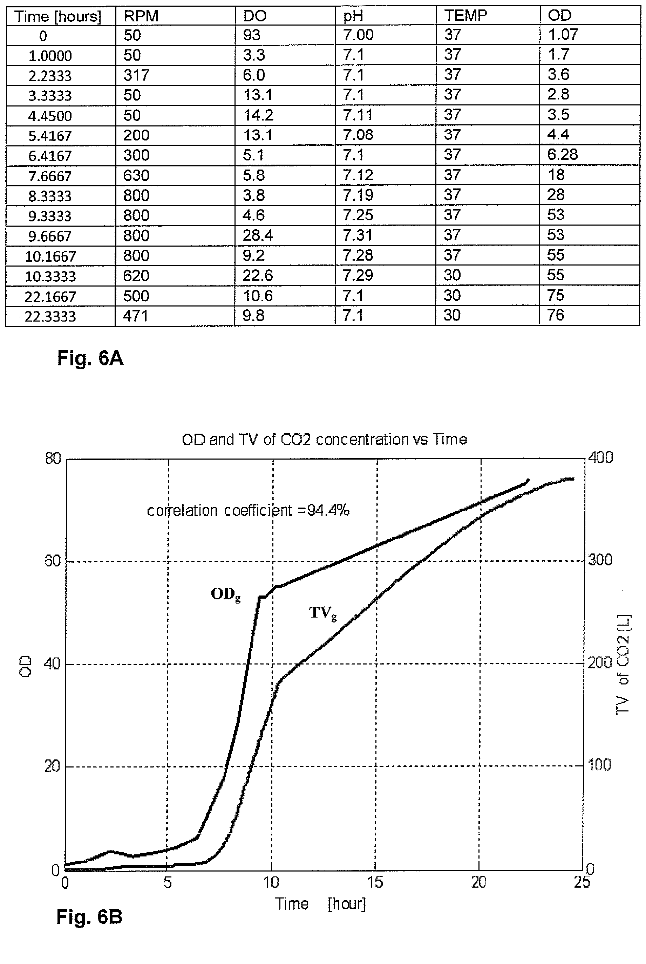

**Fig. 6A** is a table illustrating several of the above predictor parameters measured/obtained during an experiment of a fermentation process/run of recombinant protein production utilizing *Escherichia coli E.Coli* fermentation. The recombinant proteins are widely used in biotechnology and medical applications as vaccines and protein therapeutics, and as industrial enzymes for detergents and fuel ethanol production. Such recombinant protein products are made by inserting the gene that encodes the desired protein into a host cell (bacteria, yeast, insect, or animal cells) capable of producing this protein. **Fig. 6B** shows graphs **OD$_g$** and **TV$_g$** illustrating respectively the measured optical density - OD and the total volume of $CO_2$ emission-TV, as a function of time. OD and TV were measured during a fermentation experiment. As shown the OD is increased from 1 to about 55 during the first 10 hours of the experiment. Then it gradually continues to grow up to values of 75 during the next 12 hours of experiment. TV was measured continuously while OD at 15 time points as specified in the table in **Fig. 6A.** The correlation coefficient between OD and TV is 94.4%.

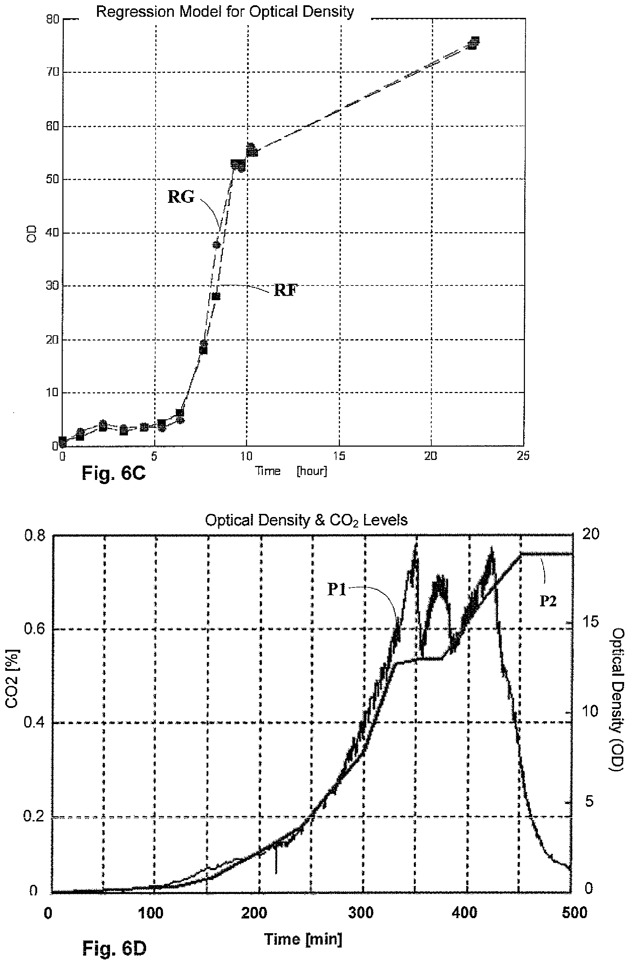

**Fig. 6C** is a graphical illustration showing two regression and reference plots, **RG** and **RF,** corresponding to the optical density of a biological material as a function of time. Plot **RF** is a reference plot obtained by direct measurements of the optical density taken during the experimental fermentation run. The regression plot **RG** is obtained during the experimental fermentation run by processing the measured $CO_2$ concentration based on the regression model which is used according to the present invention. As shown in the graphs the OD results obtained by the regression model based on the $CO_2$ concentrations are similar and almost same as those obtained by direct OD measurements.

The parameters of the regression model are estimated by means of the *robust regression* algorithm based on equations 13 and 14 as described above. More specifically, the *robust regression* operates by assigning a weight to each data point. Weighting is done automatically and iteratively using a process known as iteratively reweighted least squares. In the first iteration, each point is assigned equal weight and model coefficients ($\beta_i$ in equation 13 above) are estimated using ordinary least squares. At subsequent iterations, weights are recomputed, so that points, which were farther from model predictions in the previous iteration, are given lower weight. The model coefficients are then recomputed using weighted least squares. The process continues until the values of the coefficient estimates converge within a specified tolerance.

The regression model used in this experiment utilizes the following regression coefficients $\beta_i$ between response variable OD (*y* in equation 13 above) and the regressor/predictor variables ($x_i$ in equation (13) above) including: C, TV, RPM, pH and TEMP. To this end the following regression model was used for the estimated model parameters for prediction of OD from the measured values of $CO_2$ concentration and additional measured parameters of the fermentation process (the estimated OD being indicative of the biomass amount):

$$OD=0.38+0.17*TV+2.06*C+0.0013*(RPM-50)+22.07*(pH-7.0)-0.95*(TEMP-37) \qquad (15)$$

Fig. 6D shows the experimental results for a measured plot PI of $CO_2$ concentration (left y axis) and the plot P2 of OD (right y axis) as a function of time. As shown in the figure there is a correlation between the OD and $CO_2$ measurements through during first 325 minutes of the experiment. Then, due to stress in carbon source, the bacteria/microorganisms keep growing with alternative metabolic cycles, as seen in $CO_2$ concentration plot P1. When the biomass/microorganisms die, the $CO_2$ decreases while the OD parameter stays constant.

Thus, the present invention provides an effective and simple technique for accurate in-situ real time non-invasive monitoring of a biological material by monitoring metabolic gases in the dead space associated with the biological material. The biological material that can be monitored/inspected utilizing the invention includes but is not limited to sugars, proteins, or nucleic acids, or a combination of these substances. They may also be living entities, such as cells and tissues. They may be made from a variety of natural resources - human, animal, and microorganism-and may be produced by biotechnology methods. Example of biological product is blood transfusion products such as RBC, platelets and plasma. The biological materials may include food product (food microbiology products). Bacterial viability determination is one of the major concerns in the food industry because injured bacteria cause a significant health threat if they revive during food distribution and storage and it is important to examine the efficacy of various intervention treatments used in food processing. Also, the invention provides for effective monitoring of a fermentation process, where microorganisms are exploited to produce a wide variety of products such as dairy products (cheese, yogurt), beverages (beer, wine), single cell proteins (SCP), antibiotics, chemicals (citric and acetic acid, amino acids, enzymes, vitamins), fuels (ethanol, methanol, methane).

**Claims**

1. A method for measuring the concentrations of at least one metabolic gas emitted by microorganisms or living cells in biological material in a container, the method comprising:

   - applying non-invasive in-situ optical measurements to a region of interest being a dead space free of a biological material and in a fluid communication with a portion of the container containing the biological material, wherein said optical measurements comprise illuminating said region of interest with light of a predetermined spectrum including at least first and second predetermined wavelengths of substantially narrow spectra corresponding to respectively an absorption peak of at least one metabolic gas and a spectral region outside the absorption peak of said at least one metabolic gas, and measuring transmission of said first and second wavelengths through said dead space; and
   - analyzing measured data of said transmission and generating data indicative of a concentration of the at least one metabolic gas in said dead space which is in the fluid communication with the biological material, said generated data being thereby indicative of microorganisms in the biological material, wherein said determining of the concentration of at least one metabolic gas comprises:

      (i) measuring IR transmission through the dead space in two or more wavelengths comprising said first and second wavelengths, said measuring comprising:

         (a) tuning a central wavelength of illuminating light each one of said two or more wavelengths,
         (b) detecting IR light in said two or more wavelengths transmitted through the dead space, and
         (c) generating measured data indicative of two or more intensity values comprising first and second intensity values corresponding to the light transmitted through the dead space in said first and second wavelengths for a given optical path defined by the optical system and the dead space; and

      (ii) processing the measured data based on an absorption model of the at least one metabolic gas, said processing comprising determining a best fit between intensity values obtained from said absorption model and said measured intensity values, to thereby determine the concentration of said at least one metabolic gas.

2. The method of claim 1, comprising: processing the data indicative of the concentration of the at least one metabolic gas utilizing equilibrium condition between a rate of generation or consumption of the at least one metabolic gas emitted by living cells or microorganisms and a rate of flow of said at least one metabolic gas into and out of the container, and obtaining data about the microorganisms in the biological material.

3. The method of claim 1 or 2, wherein a spectral width of said substantially narrow spectrum of said first wavelength overlaps and exceeds a spectral width of an absorption line of said metabolic gas.

4. The method of anyone of claims 1 to 3, wherein a spectral width of said substantially narrow spectrum of said first wavelength is less than a spectral distance between two spectrally adjacent absorption lines of said metabolic gas.

5. The method of anyone of the preceding claims, wherein said dead space is defined by one of the following: (i) a portion of said container above said portion containing the biological material; (ii) a gas chamber configured to be connectable to said container so as to be in the fluid communication with said closed container; (iii) an extension of said dead space by an attached reservoir transparent to said at least first and second wavelengths.

6. The method of anyone of the preceding claims, wherein said dead space is defined by a gas chamber configured to be connectable to said container so as to be in the fluid communication with said closed container and wherein said gas chamber which is connectable to said container operates as a gas outlet thereof.

7. The method of anyone of the preceding claims, wherein said container is configured for use in a process of fermentation.

8. The method of claim 7, wherein said optical measurements and the data analysis are performed continuously or periodically, the determined gas concentration data being thereby indicative of at least one of (a) amount of microorganisms in said container as a function of time; and (b) a rate of change in amount of microorganisms in said container as a function of time; the method further comprising processing of said gas concentration data to monitor said fermentation process.

9. The method of anyone of the preceding claims, further comprising utilizing said concentration of said metabolic gas and estimating a degree of contamination of the biological material.

10. A system for use in detection of micro organisms or living cells in a biological material, the system comprising:
   an optical system comprising:

   a broadly tunable coherent IR light source configured and operable for producing light in a predetermined substantially narrow spectrum including at least first and second predetermined wavelengths corresponding to respectively an absorption peak of at least one metabolic gas and a spectral region outside the absorption peak of said at least one metabolic gas;
   a detection module comprising a detector sensitive in the IR wavelength regime, said detection module being configured for detecting light of said first and second wavelengths passing through a region of interest being a dead space free of a biological material and in a fluid communication with a portion of the container containing the biological material and located in between said light source and said detection module, and for generating data indicative of transmission of said region of interest to said at least first and second wavelengths; and
   a control system connectable to said light source and said detection module and containing functions for carrying out the following:

   - operating said light source to produce said light of at least the first and second wavelengths, selected such that the detected transmission for the first wavelength provides measured data indicative of absorbance by said at least one metabolic gas in said region of interest and the detected transmission for the second wavelength provides detection of measured reference data indicative of absorbance of said first wavelength by materials in said region of interest other than said at least one metabolic gas;
   - receiving and analyzing the measured data and the measured reference data, generating data indicative of a concentration of the at least one metabolic gas in said region of interest, enabling non-invasive in-situ detection of microorganisms in a biological material when located in fluid communication with the region of interest, wherein said determining of the concentration of at least one metabolic gas comprises:

      (i) measuring IR transmission through the dead space in two or more wavelengths comprising said first and second wavelengths, said measuring comprising:

         (a) tuning a central wavelength of illuminating light each one of said two or more wavelengths,
         (b) detecting IR light in said two or more wavelengths transmitted through the dead space, and
         (c) generating measured data indicative of two or more intensity values comprising first and second intensity values corresponding to the light transmitted through the dead space in said first and second wavelengths for a given optical path defined by the optical system and the dead space; and

      (ii) processing the measured data based on an absorption model of the at least one metabolic gas, said processing comprising determining a best fit between intensity values obtained from said absorption model and said measured intensity values to thereby determine the concentration of said at least one metabolic gas.

11. The system of claim 10 wherein, said broadly tunable light source has a tunability range of at least 2 cm$^{-1}$.

12. The system of anyone of claims 10 to 11, wherein said control system is adapted for carrying out the following:

processing the data indicative of the concentration of the at least one metabolic gas utilizing an equilibrium condition between a rate of generation or consumption of the at least one metabolic gas emitted by living cells or microorganisms and a rate of flow of said at least one metabolic gas into and out of a dead space located in the region of interest, said dead space being free of the biological material and being in fluid communication with a portion of a container in which the biological material is located; and

obtaining data about the microorganisms and/or living cells in the biological material.

13. The system according to claim 12, configured for use with a fermentation system, wherein said controller is adapted to determine the equilibrium condition based on a difference between the concentration of said at least one metabolic gas in a gas inlet to a container of the fermentation system and at least one of the following: gas contained in a container of the fermentation system; and a gas outlet flow from a container of the fermentation system; said difference corresponding to the amount of microorganisms in the container.

14. The system of claim 12, wherein the container is permeable to said at least one metabolic gas, the control system being adapted to determine the concentration of the at least one metabolic gas based on diffusion of said at least one metabolic gas through walls of said container.

15. The system of claim 13, wherein the system is configured for detecting microorganisms in a storage container for platelets and wherein said control system is adapted to perform continuous or periodical detection of the microorganisms in the region of interest to obtain gas concentration data indicative of least one of (1) amount of the gas concentration and accordingly of microorganisms in the associated biological material, as a function of time; and (2) a rate of change of amount of the gas concentration and accordingly of microorganisms in the associated biological material, and to process said gas concentration data to monitor a process of fermentation occurring in the biological material.

16. The system of anyone of claims 10 to 15, wherein said at least first and second wavelengths are in a spectral regime of high absorbance by carbon dioxide, and wherein said first wavelength overlaps one of absorption peaks of carbon dioxide in said regime and said second wavelength overlaps a transmission peak in the carbon dioxide spectrum.

**Patentansprüche**

1. Verfahren zum Messen der Konzentrationen von mindestens einem Stoffwechselgas, das von Mikroorganismen oder lebenden Zellen in biologischem Material in einem Behälter emittiert wird, wobei das Verfahren umfasst:

- Anwenden nicht-invasiver optischer In-situ-Messungen auf einen interessierenden Bereich, der ein Totraum ist, der frei von biologischem Material ist und in Fluidverbindung mit einem Teil des Behälters steht, der das biologische Material enthält, wobei die optischen Messungen das Beleuchten des interessierenden Bereichs mit Licht eines vorbestimmten Spektrums aufweist, das zumindest eine vorbestimmte erste und zweite Wellenlänge von im Wesentlichen engen Spektren aufweist, wobei die Wellenlängen jeweils einem Absorptionspeak von zumindest einem Stoffwechselgas und einem Spektralbereich außerhalb des Absorptionspeaks des zumindest einen Stoffwechselgases entsprechen, und Messen der Transmission der ersten und zweite Wellenlänge durch den Totraum; und

- Analysieren von gemessenen Daten der Transmission und Erzeugen von Daten, die hinsichtlich einer Konzentration des mindestens einen Stoffwechselgases in dem Totraum indikativ sind, der in Fluidverbindung mit dem biologischen Material steht, wobei die erzeugten Daten dadurch indikativ hinsichtlich Mikroorganismen in dem biologischen Material sind, wobei das Bestimmen der Konzentration mindestens eines Stoffwechselgases aufweist:

(i) Messen der IR-Transmission durch den Totraum bei zwei oder mehr Wellenlängen, die die erste und die zweite Wellenlänge aufweisen, wobei das Messen aufweist:

(a) Einstellen einer zentralen Wellenlänge des Beleuchtungslichts für jede der zwei oder mehr Wellenlängen,

(b) Erfassen von IR-Licht in den zwei oder mehr Wellenlängen, die durch den Totraum übertragen werden, und

(c) Erzeugen von gemessenen Daten, die in Hinsicht auf zwei oder mehr Intensitätswerte indikativ sind, die einen ersten und einen zweiten Intensitätswert aufweisen, die dem Licht entsprechen, das durch den Totraum in der ersten und der zweiten Wellenlänge für einen gegebenen optischen Weg übertragen wird, der durch das optische System und den Totraum definiert ist; und

(ii) Verarbeiten der gemessenen Daten basierend auf einem Absorptionsmodell des mindestens einen Stoffwechselgases, wobei das Verarbeiten das Bestimmen einer besten Übereinstimmung zwischen aus dem Absorptionsmodell erhaltenen Intensitätswerten und den gemessenen Intensitätswerten aufweist, um dadurch die Konzentration des mindestens einen Stoffwechselgases zu bestimmen.

2. Verfahren nach Anspruch 1, aufweisend: Verarbeiten der Daten, die indikativ für die Konzentration des mindestens einen Stoffwechselgases sind, unter Verwendung einer Gleichgewichts-Bedingung zwischen einer Erzeugungs- oder Verbrauchsrate des mindestens einen Stoffwechselgases, das von lebenden Zellen oder Mikroorganismen emittiert wird, und einer Durchflussrate des mindestens einen Stoffwechselgases in den und aus dem Behälter, und Erhalten von Daten über die Mikroorganismen in dem biologischen Material.

3. Verfahren nach Anspruch 1 oder 2, wobei eine spektrale Breite des im Wesentlichen engen Spektrums der ersten Wellenlänge eine spektrale Breite einer Absorptionslinie des Stoffwechselgases überlappt und überschreitet.

4. Verfahren nach einem der Ansprüche 1 bis 3, wobei eine spektrale Breite des im Wesentlichen engen Spektrums der ersten Wellenlänge geringer ist als ein spektraler Abstand zwischen zwei spektral benachbarten Absorptionslinien des Stoffwechselgases.

5. Verfahren nach einem der vorhergehenden Ansprüche, wobei der Totraum durch eines der folgenden Merkmale definiert ist: (i) einen Bereich des Behälters über dem Bereich, der das biologische Material enthält; (ii) eine Gaskammer, die konfiguriert ist, dass diese mit dem Behälter verbunden werden kann, um in Fluidverbindung mit dem geschlossenen Behälter zu stehen; (iii) eine Verlängerung des Totraums durch ein angebrachtes Reservoir, das für die mindestens erste und die zweite Wellenlänge transparent ist.

6. Verfahren nach einem der vorhergehenden Ansprüche, wobei der Totraum durch eine Gaskammer definiert ist, die so konfiguriert ist, dass sie mit dem Behälter verbunden werden kann, um in Fluidverbindung mit dem geschlossenen Behälter zu stehen, und wobei die Gaskammer, die mit dem Behälter verbunden werden kann, als ein Gasauslass desselben arbeitet.

7. Verfahren nach einem der vorhergehenden Ansprüche, wobei der Behälter zur Verwendung in einem Fermentationsprozess konfiguriert ist.

8. Verfahren nach Anspruch 7, wobei die optischen Messungen und die Datenanalyse kontinuierlich oder periodisch durchgeführt werden, wobei die ermittelten Gaskonzentrationsdaten dadurch indikativ für mindestens eine der folgenden Größen sind: (a) Menge von Mikroorganismen in dem Behälter als Funktion der Zeit; und (b) eine Änderungsrate der Menge von Mikroorganismen in dem Behälter als Funktion der Zeit; wobei das Verfahren ferner das Verarbeiten der Gaskonzentrationsdaten umfasst, um den Fermentationsprozess zu überwachen.

9. Verfahren nach einem der vorhergehenden Ansprüche, ferner aufweisend das Verwenden der Konzentration des Stoffwechselgases und das Schätzen eines Verschmutzungsgrades des biologischen Materials.

10. System zur Verwendung beim Nachweis von Mikroorganismen oder lebenden Zellen in einem biologischen Material, wobei das System aufweist:
ein optisches System, aufweisend:

eine breit abstimmbare kohärente IR-Lichtquelle, die zur Erzeugung von Licht in einem vorbestimmten, im Wesentlichen engen Spektrum konfiguriert und betreibbar ist, das mindestens eine erste und eine zweite vorbestimmte Wellenlänge aufweist, die jeweils einem Absorptionspeak von mindestens einem Stoffwechselgas und einem Spektralbereich außerhalb des Absorptionspeaks von mindestens einem Stoffwechselgas entsprechen;
ein Detektionsmodul mit einem Detektor, der im IR-Wellenlängenbereich empfindlich ist, wobei das Detektionsmodul zum Detektieren von Licht der ersten und zweiten Wellenlänge konfiguriert ist, das durch einen interessierenden Bereich geht, der ein Totraum ist, der frei von biologischem Material ist und in Fluidverbindung mit einem Bereich des Behälters steht, der das biologische Material enthält und sich zwischen der Lichtquelle und dem Detektionsmodul befindet, und Erzeugen von Daten, die indikativ für die Transmission des interessierenden Bereichs in Bezug auf die mindestens erste und die zweite Wellenlänge sind; und
ein Überwachungssystem, das an die Lichtquelle und das Detektionsmodul anschließbar ist und Funktionen zur Durchführung der folgenden Aufgaben aufweist:

- Betreiben der Lichtquelle, um das Licht mit mindestens der ersten und der zweiten Wellenlänge zu erzeugen, die derart ausgewählt sind, dass die detektierte Transmission für die erste Wellenlänge Messdaten liefert, die indikativ für die Absorption durch das mindestens eine Stoffwechselgas in dem interessierenden Bereich sind und die detektierte Transmission für die zweite Wellenlänge eine Detektion von gemessenen Referenzdaten liefert, die für die Absorption der ersten Wellenlänge durch Materialien in dem interessierenden Bereich, die nicht das mindestens eine Stoffwechselgas ist, indikativ sind;
- Empfangen und Analysieren der gemessenen Daten und der gemessenen Referenzdaten, Erzeugen von Daten, die für eine Konzentration des mindestens einen Stoffwechselgases in dem interessierenden Bereich indikativ sind, um nichtinvasive in-situ-Detektion von Mikroorganismen in einem biologischen Material zu ermöglichen, wenn sich dieses in Fluidkommunikation mit dem interessierenden Bereich befindet, wobei das Bestimmen der Konzentration mindestens eines Stoffwechselgases aufweist:

(i) Messen der IR-Transmission durch den Totraum bei zwei oder mehr Wellenlängen, die die erste und die zweite Wellenlänge aufweisen, wobei das Messen aufweist:

(a) Einstellen einer zentralen Wellenlänge des Beleuchtungslichts für jede der zwei oder mehr Wellenlängen,
(b) Erfassen von IR-Licht in den zwei oder mehr Wellenlängen, die durch den Totraum übertragen werden, und
(c) Erzeugen von gemessenen Daten, die in Hinsicht auf zwei oder mehr Intensitätswerte indikativ sind, die einen ersten und einen zweiten Intensitätswert aufweisen, die dem Licht entsprechen, das durch den Totraum in der ersten und der zweiten Wellenlänge für einen gegebenen optischen Weg übertragen wird, der durch das optische System und den Totraum definiert ist; und

(ii) Verarbeiten der gemessenen Daten basierend auf einem Absorptionsmodell des mindestens einen Stoffwechselgases, wobei das Verarbeiten das Bestimmen einer besten Übereinstimmung zwischen aus dem Absorptionsmodell erhaltenen Intensitätswerten und den gemessenen Intensitätswerten aufweist, um dadurch die Konzentration des mindestens einen Stoffwechselgases zu bestimmen.

**11.** System nach Anspruch 10, wobei die breit abstimmbare Lichtquelle einen Abstimmbarkeitsbereich von mindestens 2 cm$^{-1}$ aufweist.

**12.** System nach einem der Ansprüche 10 bis 11, bei dem das Steuersystem dazu eingerichtet ist, Folgendes auszuführen:

Verarbeiten der Daten, die indikativ für die Konzentration des mindestens einen Stoffwechselgases sind, unter Verwendung einer Gleichgewichts-Bedingung zwischen einer Erzeugungs- oder Verbrauchsrate des mindestens einen Stoffwechselgases, das von lebenden Zellen oder Mikroorganismen emittiert wird, und einer Durchflussrate des mindestens einen Stoffwechselgases in den und aus dem im interessierenden Bereich befindlichen Totraum, wobei der Totraum frei von biologischem Material ist und in Fluidverbindung mit einem Bereich eines Behälters steht, in dem sich das biologische Material befindet; und
Gewinnen von Daten über die Mikroorganismen und/oder lebenden Zellen im biologischen Material.

**13.** System nach Anspruch 12, das zur Verwendung mit einem Fermentationssystem konfiguriert ist, wobei die Überwachungsvorrichtung zur Ermittlung des Gleichgewichtszustands basierend auf einer Differenz zwischen der Konzentration des mindestens einen Stoffwechselgases in einem Gaseinlass zu einem Behälter des Fermentationssystems und mindestens eines der folgenden eingerichtet ist: Gas, das in einem Behälter des Fermentationssystems enthalten ist; und einen Gasauslassstrom aus einem Behälter des Fermentationssystems; wobei die Differenz der Menge an Mikroorganismen in dem Behälter entspricht.

**14.** System nach Anspruch 12, wobei der Behälter für das mindestens eine Stoffwechselgas durchlässig ist, wobei das Überwachungssystem zur Ermittlung der Konzentration des mindestens einen Stoffwechselgases basierend auf der Diffusion des mindestens einen Stoffwechselgases durch Wände des Behälters eingestellt ist.

**15.** System nach Anspruch 13, wobei das System zur Detektion von Mikroorganismen in einem Vorratsbehälter für Blutplättchen konfiguriert ist und wobei das Überwachungssystem eingestellt ist zur Ausführung einer kontinuierlichen oder periodischen Detektion der Mikroorganismen in dem interessierenden Bereich, um Gaskonzentrations-Daten zu erhalten, die für zumindest einen des Folgenden indikativ sind: (1) Betrag der Gaskonzentration und dementsprechend der Mikroorganismen im zugeordneten biologischen Material als Funktion der Zeit; und (2) eine Änderungsrate des Betrags der Gaskonzentration und dementsprechend der Mikroorganismen in dem zugehörigen biologischen Material, und zum Verarbeiten der Gaskonzentrationsdaten zum Überwachen eines Fermentationsprozesses, der in dem biologischen Material auftritt.

**16.** System nach einem der Ansprüche 10 bis 15, wobei sich die zumindest erste und zweite Wellenlänge in einem Spektralbereich mit großer Absorption durch Kohlendioxid befinden, und wobei die erste Wellenlänge einen der Absorptionspeaks von Kohlendioxid in dem Bereich und die zweite Wellenlänge einen Transmissionspeak im Kohlendioxid-Spektrum überlappt.

## Revendications

**1.** Un procédé pour mesurer les concentrations d'au moins un gaz métabolique émis par des microorganismes ou des cellules vivantes dans du matériau biologique dans un conteneur, le procédé comprenant :

- appliquer des mesures optiques in-situ non invasives à une région d'intérêt étant un espace mort sans un matériau biologique et en communication fluidique avec une portion du conteneur contenant le matériau biologique, où lesdites mesures optiques comprennent illuminer ladite région d'intérêt avec de la lumière d'un spectre prédéterminé comprenant au moins une première et une deuxième longueur d'onde prédéterminée de spectres essentiellement étroits correspondant respectivement à un pic d'absorption d'au moins un gaz métabolique et une région spectrale hors du pic d'absorption dudit au moins un gaz métabolique, et mesurer la transmission desdites premières et deuxièmes longueurs d'onde à travers dudit espace mort ; et
- analyser des dates mesurées de ladite transmission et générer des dates révélatrices d'une concentration dudit au moins un gaz métabolique dans ladite espace morte qui est en communication fluidique avec le matériau biologique, lesdites dates générées étant ici révélatrices de microorganismes dans le matériau biologique, où ladite détermination de la concentration d'au moins un gaz métabolique comprend :

(i) mesurer la transmission IR à travers l'espace mort dans une ou plusieurs longueurs d'onde comprenant ladite première et deuxième longueur d'onde, la mesure comprenant :

(a) régler une longueur d'onde centrale de la lumière d'illumination pour chacune des deux ou plusieurs longueurs d'onde,
(b) détecter de la lumière IR dans lesdites premières et deuxièmes longueurs d'onde transmise à travers l'espace mort, et
(c) générer des dates mesurées révélatrices de deux ou plusieurs valeurs d'intensité comprenant des premières et deuxièmes valeurs d'intensité correspondant à la lumière transmise à travers l'espace mort dans lesdites premières et deuxièmes longueurs d'onde pour un trajet optique donné défini par le système optique et l'espace mort ; et

(ii) traiter les données mesurées sur la base d'un modèle d'absorption dudit au moins un gaz métabolique, le traitement comprenant la détermination d'un meilleur ajustement de valeurs d'intensité obtenues dudit modèle d'absorption et desdites valeurs d'intensité mesurées pour déterminer ainsi la concentration dudit au moins un gaz métabolique.

**2.** Le procédé selon la revendication 1, comprenant : traiter les données révélatrices de la concentration dudit au moins un gaz métabolique en utilisant une condition d'équilibre entre une vitesse de génération ou de consommation dudit au moins un gaz métabolique émis par des cellules vivantes ou des microorganismes et une vitesse du flux dudit au moins un gaz métabolique dans le conteneur et hors du conteneur, et obtenir des données pour les microorganismes dans le matériau biologique.

**3.** Le procédé selon les revendications 1 ou 2, où une largeur spectrale dudit spectre essentiellement étroit de ladite première longueur d'onde chevauche et excède une largeur spectrale d'une ligne d'absorption dudit gaz métabolique.

**4.** Le procédé selon une quelconque des revendications 1 à 3, où une largeur spectrale dudit spectre essentiellement étroit de ladite première longueur d'onde est inférieure à une distance spectrale entre deux lignes d'absorption spectralement adjacentes dudit gaz métabolique.

**5.** Le procédé selon une quelconque des revendications précédentes, où ledit espace mort est défini par un des suivants : (i) une partie dudit conteneur au-dessus de ladite partie comprenant le matériau biologique ; (ii) une chambre de gaz configurée pour être connectable audit conteneur pour être en communication fluidique avec ledit conteneur fermé ; (iii) une extension dudit espace mort par un réservoir attaché transparent auxdites premières et deuxièmes longueurs d'onde.

**6.** Le procédé selon une quelconque des revendications précédentes, où ledit espace mort est défini par une chambre de gaz configurée pour être connectable audit conteneur pour être en communication fluidique avec ledit conteneur fermé et où ladite chambre de gaz qui est connectable audit conteneur fonctionne comme une sortie de gaz de celui-ci.

**7.** Le procédé selon une quelconque des revendications précédentes, où ledit conteneur est configuré pour l'utilisation dans un processus de fermentation.

**8.** Le procédé selon la revendication 7, où lesdites mesures optiques et l'analyse des données sont effectués en continue ou périodiquement, les données de concentration de gaz étant ainsi révélatrices d'au moins un parmi (a) une quantité de microorganismes dans ledit conteneur en fonction du temps ; et (b) un taux de changement de la quantité de microorganismes dans ledit conteneur en fonction du temps ; le procédé comprenant en outre le traitement des données de concentration de gaz pour surveiller ledit processus de fermentation.

**9.** Le procédé selon une quelconque des revendications précédentes, comprenant en outre l'utilisation de la concentration dudit gaz métabolique et estimer un degré de contamination du matériau biologique.

**10.** Un système à utiliser dans la détection de microorganismes ou de cellules vivantes dans un matériau biologique, le système comprenant :
un système optique comprenant :

une source de lumière IR cohérente très ajustable configurée et opérable pour produire de la lumière dans un spectre essentiellement étroit prédéterminé comprenant au moins une première et une deuxième longueur d'onde prédéterminée correspondant respectivement à un pic d'absorption d'au moins un gaz métabolique et à une région spectrale hors du pic d'absorption dudit au moins un gaz métabolique ;
un module de détection comprenant un détecteur sensible dans le régime de longueur d'onde IR, ledit module de détection étant configuré pour détecter de la lumière desdites premières et deuxièmes longueurs d'onde passant à travers une région d'intérêt étant un espace mort exempte de matériau biologique et en communication fluidique avec une partie du conteneur comprenant le matériau biologique et située entre ladite source de lumière et ledit module de détection, et pour générer des données révélatrices de la transmission de cette région d'intérêt auxdites premières et deuxièmes longueurs d'onde ; et
un système de contrôle connectable à la dite source de lumière et audit module de détection et comprenant des fonctions pour effectuer le suivant :

- faire fonctionner ladite source de lumière pour produire ladite lumière au moins desdites premières et deuxièmes longueurs d'onde choisies de sorte que la transmission détectée pour la première longueur d'onde fournit des données mesurées révélatrices d'absorption par ledit au moins un gaz métabolique dans ladite région d'intérêt et que la transmission détectée pour la deuxième longueur d'onde prévoit la détection de données de référence mesurées révélatrices de l'absorption de ladite première longueur d'onde par des matériaux dans ladite région d'intérêt différentes dudit au moins un gaz métabolique ;
- recevoir et analyser les données mesurées et les données de référence mesurées, générer des données indiquant une concentration dudit au moins un gaz métabolique dans ladite région d'intérêt pour permettre une détection non-invasive in-situ de microorganismes dans un matériau biologique lors d'un arrangement en communication fluidique avec la région d'intérêt, où ladite détermination de la concentration d'au moins un gaz métabolique comprend :

(i) mesurer la transmission IR à travers l'espace mort dans une ou plusieurs longueurs d'onde comprenant lesdites premières et deuxièmes longueurs d'onde, la mesure comprenant :

(a) ajuster une longueur d'onde centrale de lumière d'illumination à chacune des deux ou plusieurs longueurs d'onde,

(b) détecter de la lumière IR dans lesdites deux ou plusieurs longueurs d'onde transmise à travers l'espace mort, et

(c) générer des dates mesurées révélatrices de deux ou plusieurs valeurs d'intensité comprenant une première et une deuxième valeur d'intensité correspondant à la lumière transmise à travers l'espace mort dans lesdites premières et deuxièmes longueurs d'onde pour un trajet optique donné défini par le système optique et l'espace mort ; et

(ii) traiter les données mesurées sur la base d'un modèle d'absorption dudit au moins un gaz métabolique, le traitement comprenant la détermination d'un meilleur ajustement de valeurs d'intensité obtenues dudit modèle d'absorption et desdites valeurs d'intensité mesurées pour déterminer ainsi la concentration dudit au moins un gaz métabolique.

11. Le système selon la revendication 10, où ladite source de lumière très ajustable a une gamme d'ajustement d'au moins 2 cm$^{-1}$.

12. Le système selon une quelconque des revendications 10 à 11, où ledit système de contrôle est adapté pour effectuer le suivant :

traiter les données révélatrices de la concentration dudit au moins un gaz métabolique en utilisant une condition d'équilibre entre une vitesse de production ou de consommation dudit au moins un gaz métabolique émis par des cellules vivantes ou des microorganismes et une vitesse du flux dudit au moins un gaz métabolique dans et hors d'un espace mort situé dans la région d'intérêt, ledit espace mort étant exempte de matériau biologique et en communication fluidique avec une partie du conteneur où le matériau biologique est situé ; et obtenir des données sur les microorganismes et/ou les cellules vivantes dans le matériau biologique.

13. Le système selon la revendication 12, configuré pour l'utilisation avec un système de fermentation, où ladite commande est adaptée pour déterminer la condition d'équilibre sur la base d'une différence entre la concentration dudit au moins un gaz métabolique dans une entrée de gaz à un conteneur du système de fermentation et au moins un des suivants : du gaz contenu dans un conteneur du système de fermentation, et un flux de sortie de gaz d'un conteneur du système de fermentation ; ladite différence correspondant à la quantité de microorganismes dans le conteneur.

14. Le système selon la revendication 12, où le conteneur est perméable audit au moins un gaz métabolique, le système de commande étant configuré pour déterminer la concentration dudit au moins un gaz métabolique sur la base de la diffusion à travers des parois dudit conteneur.

15. Le système selon la revendication 13, où le système est configuré pour détecter des microorganismes dans un conteneur de stockage pour des plaquettes et où ledit système de commande est configuré pour effectuer une détection continue ou périodique des microorganismes dans la région d'intérêt pour obtenir des données de concentration de gaz révélatrices d'au moins un parmi (1) quantité de la concentration de gaz et en conséquence de microorganismes dans le matériau biologique associé en fonction du temps ; et (2) un taux de changement de la quantité de la concentration de gaz et en conséquence de microorganismes dans le matériau biologique associé et pour traiter les données de concentration de gaz pour surveiller un processus de fermentation ayant lieu dans le matériau biologique.

16. Le système selon une quelconque des revendications 10 à 15, où lesdites au moins premières et deuxièmes longueurs d'onde sont dans une gamme spectrale de haute absorption par du dioxyde de carbone, et où ladite première longueur d'onde chevauche un des pics d'absorption de dioxyde de carbone dans ladite gamme et où ladite deuxième longueur d'onde chevauche un pic de transmission dans le spectre de dioxyde de carbone.

**Fig.1A**

**Fig. 1B**

**Fig. 1C**

**Fig. 1D**

**Fig. 1E**

**Fig. 1F**

**Fig. 2A**

**Fig. 2B**

**Fig. 3**

QCL Absorbed Intensity vs wavelength and concentration of CO2 for 8 cm path at Normal pressure

**Fig. 4**

**Fig. 5A**

**Fig. 5B**

| Time [hours] | RPM | DO | pH | TEMP | OD |
|---|---|---|---|---|---|
| 0 | 50 | 93 | 7.00 | 37 | 1.07 |
| 1.0000 | 50 | 3.3 | 7.1 | 37 | 1.7 |
| 2.2333 | 317 | 6.0 | 7.1 | 37 | 3.6 |
| 3.3333 | 50 | 13.1 | 7.1 | 37 | 2.8 |
| 4.4500 | 50 | 14.2 | 7.11 | 37 | 3.5 |
| 5.4167 | 200 | 13.1 | 7.08 | 37 | 4.4 |
| 6.4167 | 300 | 5.1 | 7.1 | 37 | 6.28 |
| 7.6667 | 630 | 5.8 | 7.12 | 37 | 18 |
| 8.3333 | 800 | 3.8 | 7.19 | 37 | 28 |
| 9.3333 | 800 | 4.6 | 7.25 | 37 | 53 |
| 9.6667 | 800 | 28.4 | 7.31 | 37 | 53 |
| 10.1667 | 800 | 9.2 | 7.28 | 37 | 55 |
| 10.3333 | 620 | 22.6 | 7.29 | 30 | 55 |
| 22.1667 | 500 | 10.6 | 7.1 | 30 | 75 |
| 22.3333 | 471 | 9.8 | 7.1 | 30 | 76 |

## Fig. 6A

Fig. 6B

Fig. 6C

Fig. 6D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5155019 A
- US 4889992 A
- US 5482842 A
- EP 1724335 A